# EUROPEAN PATENT SPECIFICATION

(11) **EP 1 390 491 B1**
(45) Date of publication and mention of the grant of the patent: **16.07.2008**
(21) Application number: 02769340.7
(22) Date of filing: 02.05.2002
(51) Int. Cl.: C12N 15/00, C07K 14/47, A01K 67/033, A61K 31/167, A61K 31/19, A61K 31/192, A61K 31/4406, A61P 25/00

(54) **METHOD FOR TREATING NEURODEGENERATIVE, PSYCHIATRIC AND OTHER DISORDERS WITH DEACETYLASE INHIBITORS**
VERFAHREN ZUR BEHANDLUNG NEURODEGENERATIVER, PSYCHIATRISCHER UND ANDERER STÖRUNGEN MIT DEACETYLASEINHIBITOREN
METHODE DE TRAITEMENT DES MALADIES NEURODEGENERATIVES, PSYCHIATRIQUES ET AUTRES AVEC DES INHIBITEURS DE LA DEACETYLASE

(30) Priority: 02.05.2001 US 288215 P; 11.04.2002 US 372724 P
(43) Date of publication of application: 25.02.2004
(73) Proprietor: The Regents of the University of California, Oakland, CA 94607-5200 (US)
(72) Inventor: STEFFAN, Joan, S., Laguna Beach, CA 92651 (US); THOMPSON, Leslie, M., Irvine, CA 92612 (US); MARSH, J., Lawrence, Newport Beach, CA 92660 (US); BODAI, Laszlo, Irvine, CA 92612 (US); PALLOS, Judit, Irvine, CA 92612 (US)
(74) Representative: Grünecker, Kinkeldey, Stockmair & Schwanhäusser Anwaltssozietät
(86) International application number: PCT/US2002/014167
(87) International publication number: WO 2002/090534

(56) References cited:
- WO-A-97/35990
- MARKS P A ET AL: "HISTONE DEACETYLASE INHIBITORS: INDUCERS OF DIFFERENTIATION OR APOPTOSIS OF TRANSFORMED CELLS" JOURNAL OF THE NATIONAL CANCER INSTITUTE, US DEPT. OF HEALTH, EDICATIONAND WELFARE, PUBLIC HEALTH, US, vol. 92, no. 15, 2 August 2000 (2000-08-02), pages 1210-1216, XP001121734 ISSN: 0027-8874
- MARSH J L ET AL: "Expanded polyglutamine peptides alone are intrinsically cytotoxic and cause neurodegeneration in Drosophila." HUMAN MOLECULAR GENETICS. 1 JAN 2000, vol. 9, no. 1, 1 January 2000 (2000-01-01), pages 13-25, XP002304372 ISSN: 0964-6906
- BATES G.P. ET AL.: 'Transgenic models of huntington's disease' HUMAN MOLECULAR GENETICS vol. 6, no. 10, REV. ISSUE, 1997, pages 1633 - 1637, XP002955976
- MANGIARINI L. ET AL.: 'Instability of highly expanded CAG repeats in mice transgenic for the Huntington's disease mutation' NATURE GENETICS vol. 15, no. 2, February 1997, pages 197 - 200, XP002956112
- BATES G.P. ET AL.: 'Polyglutamine expansion and Huntington's disease' BIOCHEMICAL SOCIETY TRANSACTIONS vol. 26, no. 3, 1998, pages 471 - 475, XP002957811
- SARATIKOV A.S. ET AL.: 'Action of lithium hydroxybutyrate on electro encephalographic effects of amphetamine' BULLETIN OF EXPERIMENTAL BIOLOGY AND MEDICINE vol. 94, no. 7, 1982, pages 907 - 910, XP002957801
- LEVY A.I. ET AL.: 'Gamma hydroxy butyrate in the treatment of schizophrenia' PSYCHIATRY RESEARCH vol. 9, no. 1, 1983, pages 1 - 8, XP002957802

## Description

### BACKGROUND OF THE INVENTION

Huntington's disease (HD) is an autosomal dominant, late-onset neurodegenerative disorder characterized by motor abnormalities, cognitive dysfunction, and psychiatric symptoms (Harper, P. S., ed. (1991) Huntington's Disease (W. B. Saunders, London), pp. 141-178). HD is caused by an expansion of a polyglutamine tract in the amino-terminal portion of a predominantly cytosolic protein, huntingtin (Htt) (The Huntington's Disease Collaborative Research Group (1993) Cell 72, 971-983; DiFiglia, M., et al. (1995) Neuron 14, 1075-1081). Repeat expansions greater than approximately 38 repeats cause disease, while unaffected individuals can have repeat lengths of up to 35 repeats (Gusella, J. F. & MacDonald, M. E. (1995) Semin. Cell Biol. 6, 21-28).

WO 97/35990 describes Hdx polypeptides capable of modulating proliferation , survival and/or differentiation of cells due to their ability to alter chromatin structure by deacetylating histones such as H3 or H4.

A transgenic mouse model for HD has shown that human *huntingtin* exon 1 carrying an expanded CAG repeat is sufficient to cause a HD-like phenotype (Mangiarini, L., et al. (1996) Cell 87, 493-506). Preceding onset of neurological symptoms, the appearance of intranuclear inclusions, nuclear localized aggregates containing truncated Htt and ubiquitin proteins have been found in neurons of HD transgenic mice (Davies, S. W., et al. (1997) Cell 90, 537-548). These inclusions have also been identified in neurons and dystrophic neurites in HD brain tissue (DiFiglia, M., et al. (1997) Science 277, 1990-1993) and as cytosolic aggregates in the neuronal processes (neuropil) of both HD patient and transgenic mouse brain tissues (Gutekunst, C. A., et al. (1999) J. Neurosci. 19, 2522-2534; Li, S.-H., et al. (1999) J. Neurosci. 19, 5159-5172).

Co-localization of a variety of cellular proteins, including transcription factors such as CREH-binding protein (CBP) (Kazantsev, A., et al. (1999) Proc. Natl. Acad. Sci. USA 96,11404-11409), TATA-binding protein (TBP) (Huang, C., et al. (1998) Somatic Cell Mol. Genet. 24, 217-233), and mSin3a (Boutell, J. M., et al. (1999) Hum. Mol. Genet. 9, 1647-1655), have been shown in cell culture (CBP) and human brain (TBP and mSin3a) inclusions. The role that aggregation plays in disease progression is unclear (Paulson, H. L. (1999) Am. J. Hum. Genet. 64, 339-345; Reddy, P. H., Williams, M. & Tagle, D. A. (1999) Trends Neurosci. 22, 248-255; Perutz, M. (1999) Trends Biochem. Sci. 24, 58-63); however, nuclear localization of the protein following cytosolic cleavage (Sieradzan, K., et al. (1999) Exp. Neurol. 156, 92-99) has been implicated as having an important role in disease pathogenesis in both cell culture (Saudou, F., et al. (1998) Cell 95, 55-66) and in transgenic mouse models (Klement, I. A., et al. (1998) Cell 95, 41-53).

HD results in selective neuronal degeneration, especially of the striatum and cerebral cortex (Vonsattel, J. P., et al. (1985) J. Neuropathol. Exp. Neurol. 44, 559-577). There is evidence that programmed cell death (apoptosis) may be involved in chronic neurodegenerative disorders including Alzheimer's, Parkinson's, and Huntington's diseases (Desjardins, P. & Ledoux, S. (1998) Metab. Brain Dis. 13, 79-92).

The tumor suppressor protein p53 plays a central role in determining whether a cell will undergo differentiation, senescence, or apoptosis (Levine, A. J. (1997) Cell 88, 323-331) and has been implicated in the regulation of neuronal apoptosis (Hughes, P. E., Alexi, T. & Schreiber, S. S. (1997) NeuroReport 8, 5-12). Both CBP and mSin3a interact with p53 and are involved in p53-mediated transcriptional regulation (Murphy, M., et al. (1999) Genes Dev. 13, 2490-2501).

Apoptotic cell death in HD has been observed using postmortem HD brain tissue (Petersen, A., Mani, K. & Brundin, P. (1999) Exp. Neurol. 157, 1-18) and in some mouse models (Reddy, *supra),* although the mechanism for cell death remains unclear. Currently, no cure or effective treatment for this agonizing and lethal disease exists. The etiology of several other neurodegenerative diseases, including spinocerebellar ataxia I and Kennedy's disease, also involves a polyglutamine repeat expansion (Zoghbi, H. Y. & Orr, H. T. (2000) Annu Rev Neurosci 23, 217-247). What is needed, therefore, is a method for treating patients with Huntington's disease or other polyglutamine-repeat and/or neurodegenerative diseases, to either prevent or suppress neurodegeneration, even after onset of the pathology.

### SUMMARY OF THE INVENTION

The invention relates to a novel method for treating a variety of diseases and disorders, including neurological degeneration, psychiatric disorders, aggregation disorders and diseases arising from polyglutamine expansion, such as Huntington's disease, comprising administering to patients in need thereof a therapeutically effective amount of one or more deacetylase inhibitors.

In addition to Huntington's disease, the administration of deacetylase inhibitors according to practice of the present invention may be used to treat Kennedy's disease, spinocerebellar ataxia, dentatorubral-pallidoluysian atrophy (DRPLA), Machado-Joseph disease, Alzheimer's disease, amyotrophic lateral sclerosis, Parkinson's disease, schizophrenia, and prion-related diseases. Other diseases and disorders, including those arising from polyglutamine expansion, may also be treated by deacetylase inhibitors as will be appreciated by the skilled practitioner.

The invention is also directed to a transgenic fly useful as a model of polyglutamine expansion diseases, which may be used to test potential therapeutic agents.

### BRIEF DESCRIPTION OF THE DRAWINGS

**FIG. 1****.** Schematic representation of the interaction between histone deacetylase complexes and acetyltransferase proteins, with and without inhibitors of HDAC inhibitors, in regulating gene transcription.
**FIG. 2**. Western blot of solubilized proteins purified from aggregates generated in HEK293 cells expressing 103Q-GFP.
**FIG. 3****.** Autoradiogram of SDS-PAGE gel showing binding of [³⁵S]methionine-labeled p53(1-393) and p53(1-347) to GST-Htt fusion proteins.
**FIG. 4**. Western blot using monoclonal anti-p53 antibody, showing co-precipitation of p53 with Httex1p in HEK293 cells.
**FIG. 5****.** Bar graph showing repression of transcription by expanded Httex 1p.
**FIG. 6****.** Bar graph showing that huntingtin represses p21/WAF1/Cip 1 transcription in stable PC12 cells.
**FIG. 7****.** Autoradiogram of SDS-PAGE gels showing binding of[³⁵S]methionine-labeled CBP (I) or mSin3a (II) to GST-Htt fusion proteins.
**FIG. 8**. Stained striatal sections showing the localization of CBP to neuronal intranuclear inclusions in R6/2 mice. Striatal sections from R6/2 (A-C) and littermate controls (D) were iminunolabeled with anti-Htt (A), anti-ubiquitin (B), and anti-CBP (C and D) antibodies. Nuclei were counterstained with methyl green. Nuclear inclusions are indicated by arrows. Scale bar, 15 µm.
**FIG. 9****.** Schematic representations of CREB-binding protein (CBP) and p300/CBP-associated factor (P/CAF). RID, nuclear hormone receptor interacting domain; CH 1, CH2, CH3, cysteine-histidine-rich regions 1, 2, and 3; Br, bromodomain; KIX, CREB-binding domain. The amino acid residues used as ³⁵S-labeted probes for GST pull-down assays with GST-Htt proteins are designated below each protein diagram.
**FIG. 10****.** Graph showing the results of GST pull-down assays using radioactive domains of CBP and P/CAF with GST-51QP Htt. The acetyltransferase (AT) domains of both CBP and P/CAF interact with GST-51QP.
**FIG. 11****.** Interaction of GST-Htt fusion proteins, containing wild-type and expanded polyglutamine stretches, with and without the proline-rich domain, with radioactive probes containing the AT domains of CBP and P/CAF. A representative autoradiogram of the middle panel is shown. The slight alteration in the pattern ofCBP(1,459-1,877) in the GST-51QP lane as compared with the other lanes is due to co-migration of GST-51QP with labeled CBP(1,459-18,77).
**FIG. 12****.** Graphs showing inhibition ofthe acetyl-transferase activityof CBP, p300 and P/CAF by Htt *in vitro*. GST-fusion proteins GST-20QP, GST-51 QP, and GST-51 Q inhibit the acetyltransferase activity of GST-CBP (amino acids 1099-1877), GST-p300 (amino acids 1195-1707) and GST-P/CAF (amino acids 87-832) on biotinylated H4 peptide.
**FIG. 13. (a)** PC12 cells induced to express 25QP-GFP or 103QP-GFP show a reduction in the level of acetylation of both histones H3 and H4. **(b)** A second strain of stably transfected PC12 cells, grown in the absence ofbutyrate but induced to express F103QP-EGFP, shows a reduction in the level of acetylation of histone H4, which can be reversed by butyrate treatment. Equivalent levels of histones are shown by Coomassie-blue staining (**a** and **b**) or with anti-histone H3 (a only).
**FIG. 14****.** Graph showing the progressive loss in the number of rhabdomeres per ommatidium at 1 and 6 days after eclosion in flies expressing Httexlp Q93.
**FIG. 15****.** Graph showing that administration of SAHA slows photoreceptor degeneration. The number of rhabdomeres per ommatidium at 6 days after eclosion is markedly improved in Httexlp Q93 expressing flies fed SAHA. Animals were fed 0.5, 2 and 10 µM SAHA. Results with 2 µM SAHA are shown.
**FIG. 16****.** Graph showing that administration of butyrate slows neuronal degeneration. The number of rhabdomeres per ommatidium at 6 days after eclosion is markedly improved in Httex1p Q93 expressing flies fed butyrate. Animals were fed 10, 30 and 100 mM butyrate. Results with 100 mM butyrate are shown.
**FIG.17****.** Graph showing that administration of SAHA improves the 6 day survival of adult flies expressing Httex 1p. Animals were fed 0.5, 2 and 10 µM SAHA dissolved in DMSO. Per cent rescue was calculated as follows: (per cent surviving - per cent surviving on solvent alone)/(1 - per cent surviving on solvent alone).
**FIG. 18****.** Graph showing that genetically reducing deacetylase activity in *Sin3A* heterozygotes slows degeneration. Flies expressing Httex1p Q93 and heterozygous for a *Sin3A* mutation were compared with similar flies without the *Sin3A* mutation. The photoreceptor distribution was monitored at 6 days.
**FIG. 19****.** Western blot showing that expression of the Httexlp Q93 transgene is unchanged by treatment with either SAHA or butyrate. A Western blot of extracts from larvae expressing Httex 1p Q93 and treated either with solvent alone or solvent with SAHA or butyrate was probed with anti-Htt antibody. Similar amounts of protein were loaded, as determined by Bradford assays and confirmed by Coomassie staining of the gel (not shown).
**FIG. 20**. Graph showing that rhabdomeres in the eyes of flies expressing tagged Q48 peptides show progressive loss.
**FIG. 21**. Graph showing that progressive photoreceptor neuron degeneration is arrested by 2 µM SAHA even when feeding is initiated only in adult flies.
**FIG. 22**. Graph showing that progressive photoreceptor neuron degeneration is arrested by 100 mM butyrate even when feeding is initiated only in adult flies.
**FIG. 23****.** Photographs of ommatidia from a normal fly and from flies expressing Q48 with and without deacetylase inhibitors butyrate and SAHA.
**FIG. 24**. Graph showing the distribution of rhabdomeres per ommatidia in Sir-2 flies.
**FIG. 25**. Graph showing the distribution of rhabdomeres per ommatidia in Mi-2 flies.

### DETAILED DESCRIPTION OF THE INVENTION

**Abbreviations.** Abbreviations used herein are indicated in Table 1.

**Table 1**

| **Abbreviation** | **Meaning** |
|---|---|
| AR | Androgen receptor |
| AT | Acetyltransferase |
| Br | Bromodomain |
| CBP | CREB-binding protein |
| CH | Cysteine-histidine rich region |
| DRPLA | Dentatorubral-pallidoluysian atrophy |
| GFP | Green fluorescent protein |
| GST | Glutathione-S-transferase |
| HAT | Histone acetyltransferase |
| HD | Huntington's disease |
| HDAC | Histone deacetylase |
| Htt | Huntingtin |
| Httex 1p | Huntingtin exon 1 protein |
| KIX | CREB-binding domain |
| P/CAF | p300/CBP-associated factor |
| PMSF | phenyl-methylsulfonyl fluoride |
| Poly Q | Polyglutamine |
| RID | Nuclear hormone receptor interacting domain |
| RXRα | Retinoid X receptor alpha |
| SAHA | Suberoylanilide hydroxamic acid |
| SH3 | Src homology 3 |
| TBP | TATA-binding protein |
| TSA | Trichostatin A |
| UAS | Upstream activator sequence |

**Overview of the Invention**. Using a novel approach to evaluate the presence of cellular proteins associated with aggregates, the present inventors have demonstrated that mutant Htt exon 1 protein (Httexlp) containing an expanded polyglutamine repeat co-aggregates with p53 in cell culture-generated inclusions, and interacts with p53 in biochemical assays. The present inventors have also demonstrated that expanded Httex 1p interacts *in vitro* with two other critical transcription factors, the co-activator CBP and the co-repressor mSin3a. The present inventors have further shown that CBP localizes to neuronal intranuclear inclusions in a transgenic mouse model of HD.

In addition, the present inventors have demonstrated that Httex1p directly binds the acetyltransferase domains of two distinct proteins, CREB-binding protein (CBP) and p300/CBP-associated factor (P/CAF) and that in cell-free assays, Httex 1p also inhibits the acetyltransferase activity of at least three enzymes: p300, P/CAF and CBP. The present inventors have shown that expression of Httex1p in cultured cells reduces the level of acetylated histones H3 and H4, and that this reduction can be reversed by administering of inhibitors of histone deacetylase (HDAC). Finally, the present inventors have demonstrated that, *in vivo,* HDAC inhibitors arrest ongoing progressive neuronal degeneration induced by polyglutamine repeat expansion, and reduce lethality in two *Drosophila* models of polyglutamine disease.

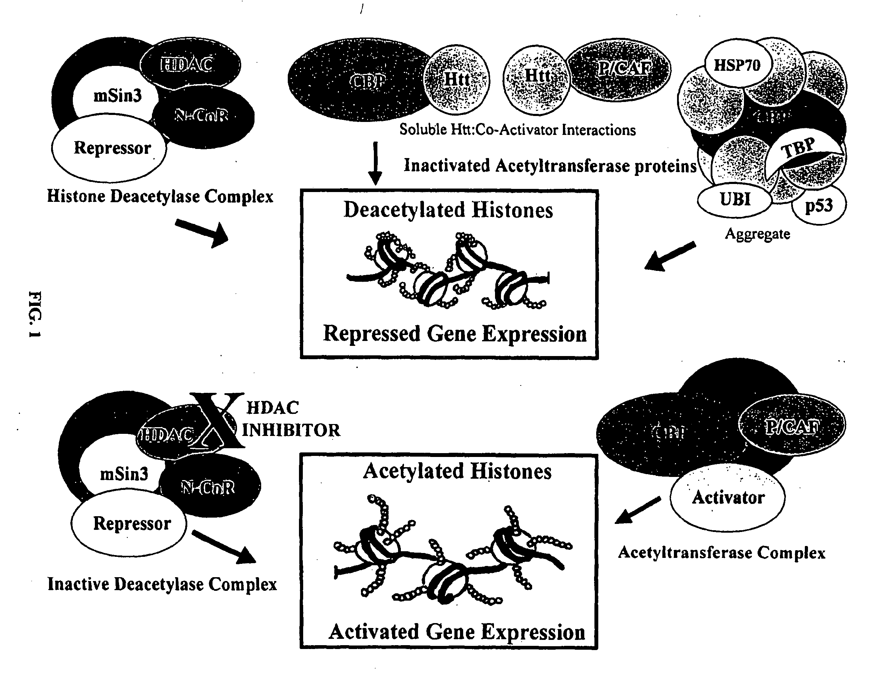

These data suggest that expanded polyglutamine repeats in the context of a disease protein may cause aberrant transcriptional regulation, leading to neuronal dysfunction and degeneration in HD and other triplet repeat diseases, by disrupting the interplay between acetyltransferase proteins and histone deacetylase complexes, resulting in a decrease in the levels of protein acetylation. A model of the interaction between histone deacetylase complexes, acetyltransferase proteins, and Htt, with and without HDAC inhibitors, is shown in FIG. 1.

The present invention is thus directed to the use of HDAC inhibitors to slow and/or prevent the progressive neurodegeneration seen in Huntington's disease and other polyglutamine repeat diseases and/or aggregation diseases, even after onset of symptoms. The present invention is further directed to the use of HDAC inhibitors to treat various psychiatric diseases and disorders, including schizophrenia, bipolar disorder and depressive illness, in which transcriptional repression has been implicated.

In addition, the present invention is directed to the use of a transgenic *Drosophila* stock as a model ofpolyglutamine disease, for the testing and evaluation of potential therapeutic agents for the treatment and/or prevention of polyglutamine diseases.

**Plasmid constructs**. Alternating CAG/CAA repeats, coding for either a normal range or expanded polyglutamine tract, were put into the context of either a truncated [first 17 amino acids plus poly(Q) repeat] or a complete Htt exon 1 (Kazantsev, A., et al. *supra)* and subcloned into pcDNA 3.1 (Invitrogen, Carlsbad, California). Repeats encoding 25 (25QP-GFP) or 103 (103QP-GFP) glutamine amino acids within the complete exon 1 sequence were fused in-frame to the coding sequence for an enhanced green fluorescence protein tag at the 3' end of each construct. Truncated exon 1 constructs were also created encoding a myc epitope tag at the carboxyl terminus instead of enhanced green fluorescence protein. Various Httexp1-polyQ constructs are described in Waelter, S. et al. (2001) Molecular Biology of the Cell, 12, 1393-1407.

GST-Htt exon 1 fusion proteins containing 20, 51, and 83 polyglutamine repeats were prepared as described in Scherzinger, E., et al. (1997) Cell 90, 549-558. Truncated GST-103Q fusion protein was derived from the 103Q-myc construct by subcloning into pGEX-3X (Amersham Biosciences, Piscataway, New Jersey), eliminating the myc coding region. pGEX-2T (Amersham Biosciences) was used for the GST control. DNA molecules encoding GST fusion proteins were ligated into pcDNA3.1 for expression in mammalian cells.

Full-length p53 (encoding amino acids 1-393), encoded within a 1.8-kb *Bam*HI fragment, was subcloned into pcDNA 6.0 *myc*/His C (Invitrogen). To create a truncated p53 (amino acids 1-347), the previous construct was digested with *Stu*I and re-ligated. cDNAs encoding either CBP in pcDNA3.1 or mSin3a in pcDNA3 were used for protein synthesis. For expression in mammalian cells, Htt exon 1 constructs encoding 20 (20QP) or 93 (93QP) polyglutamine tracts in pTL1 (Sittler, A., et al. (1998) Mol. Cell 2, 427-436.) were used.

The WAF1-luciferase fusion was created by annealing the two oligonucleotides, 5'-CGCGTGAATTCGAACATGTCCCAACATGTTGCCC-3'and 5'-GGGCAACATGTTGGGACATGTTCGAATTCA-3',containing two copies of the WAF1 p53 binding site, generating a duplex with 5' *Mlu*I and *Eco*RI sites and a 3' *Sma*I site and subcloning into the *Mlu*I*-Sma*I site of the pGL3-promoter vector (Promega, Madison, Wisconsin).

pGST-P/CAF/AT domain (containing the *Sac*II/*Nco*I fragment encoding amino acids 87-768) and pcDNA3 containing the cDNA for P/CAF (Yang, X. J., et al. (1996) Nature 382, 319-324) were also constructed. A FLAG tag was cloned in front of alternating CAG/CAA repeats to create F103QP-EGFP. For transgenic *Drosophila* lines, Htt exon 1 constructs were subcloned into pUAST, described in Brand, A. & Perrimon, N. (1993) Development 118, 401-415.

The full-length p53 was provided by Eric Stanbridge, University of California, Irvine. The mSin3A (available from Affinity BioReagents, Golden, Colorado) in pcDNA3 was provided by Maureen Murphy, Fox Chase Cancer Center, Philadelphia, Pennsylvania).

**Isolation and Biochemical Analyses of Poly(Q) Aggregates**. HEK293 cells were transiently transfected with 103 Q-GFP or 103QP-GFP encoding constructs using LIPOFECTIN® (Invitrogen) or GENEPORTER^{™} II (Gene Therapy Systems, San Diego, California). Aggregates in the media from plates of efficiently transfected cells (85-90%) showing extensive aggregate formation were pelleted by centrifugation and washed with PBS and 0.1% Triton X-100/PBS.

Aggregates were incubated with rocking for 30 min in 0.1% Triton X-100/PBS, overnight in 1% Triton X-100/PBS, 2 h in 0.1% SDS/PBS, and 30 min in 1% SDS/PBS; following each incubation step, aggregates were pelleted by centrifugation. For intact cells, 0.3% NP-40 was first used to lyse the cells.

Experiments were performed with and without 100 µM PMSF in the buffers and at room temperature or at 4°C with similar results due to the stability of the aggregates. Enrichment of aggregates was followed microscopically by monitoring GFP fluorescence.

Semipurified aggregates were boiled in 2X loading buffer containing 4% SDS, 10% β-mercaptoethanol, 10 mM DTT, 20% glycerol, 0.1 M Tris-HCl (pH 6.8), and 4 mM EDTA for 10 min. Levels of immunoreactive protein were determined for each antibody in whole-cell extracts from the transfected cells and proportionate amounts of immunoreactive protein were loaded in equivalent ratios of whole-cell extract to aggregate preparation on 8% SDS gels.

The gels were transferred overnight to Immobilon-P membrane (Millipore, Bedford, Massachusetts) by standard Western blot wet transfer methods. The immobilon membrane was blocked for 1 h in 5% Fraction V BSA (United States Biochemical, Cleveland, Ohio) in 1X TBS/Tween 20, cut into strips and incubated for 1 h in antibody, washed three times, and incubated with goat anti-mouse or goat anti-rabbit horseradish peroxidase-conjugated antibodies (The Jackson Laboratory, Bar Harbor, Maine). Antibodies used included anti-p53 (DO-1), anti-mSin3a (AK-11), anti-CBP (A-22), anti-mdm2 (SMD14), anti-RXRα (D-20), anti-NF-κB p65 (F-6), anti-AR (N-20) (all from Santa Cruz Biotechnology, Santa Cruz, California), and anti-Htt polyclonal (Q51) (Sittler, A., et al., supra).

Immunoreactive bands were detected using enhanced chemiluminescence (Amersham Biosciences). Following enhanced chemiluminescence, blots were incubated with ¹²⁵I-protein A, washed three times in TBS/Tween 20 and quantitated by phosphorimager analysis.

**GST Pull-Down Assays.** GST pull-down experiments were performed as previously described (Steffan, J. S., et al. (1998) Mol. Cell. Biol. 18, 3752-3761; Steffan, J. S., et al. (1996) Genes Dev. 10, 2551-2563). [³⁵S]Methionine-labeled full-length p53 (1-393), truncated p53 (1-347), CBP, and mSin3a were synthesized *in vitro* by using rabbit reticulocyte lysate systems (Promega). The radiolabeled proteins were added in approximately equal molar amounts to GST fusion proteins pre-equilibrated in buffer A (20 mM Hepes-KOH, pH 7.5, 100 mM NaCl, 10% glycerol, 0.1 % Triton-X-100, and 100 µM PMSF) containing 0.2% BSA in a final volume of 200 µl. After a 30-min rocking at room temperature, the beads were recovered and washed three times at room temperature in 1 ml ofbuffer A. The bound proteins were analyzed by SDS-PAGE followed by autoradiography and phosphorimager analysis.

For cell culture experiments, 2- X 100-mm dishes of HEK293 cells were transiently transfected with a total of 40 µg of pcDNA3.1 plasmids encoding the following proteins, using LIPOFECTIN® (Invitrogen): 13 µg of either GST, GST-20QP, GST-83QP, or GST 103Q, 13 µg of p53, and 14 µg of CBP. Twenty-four hours later, cells were harvested and resuspended in 400 µl of buffer A including 10 µg/ml of aprotenin and leupeptin, with 100 µl of sterile acid-washed glass beads in a 1.6-ml microfuge tube.

Cells were vortexed for 30 s and then placed on ice. Lysates were spun in a microfuge at 2000 rpm for 2 min, and supernatants were collected and assayed for protein concentration by Bradford analysis. Three hundred micrograms of lysates were incubated with 30 µl of glutathione-agarose beads (Sigma, St. Louis, Missouri) for 30 min at 4°C with rocking in Buffer A. Beads were then washed five times in 1 ml of Buffer A, resuspended in 2X Laemmli buffer, boiled 3 min, and loaded directly onto an 8% SDS-polyacrylamide gel. Western blot analysis was performed as above.

Binding and activity data were compared by an analysis of variance with StatView.

**Luciferase Assays.** For WAF1-luciferase assays, SAOS-2 cells in 6-well plates were transiently transfected by calcium phosphate precipitation using 100 ng of p53 expression vector, 2 µg of WAF1-luciferase expression vector, and 2 µg of pcDNA3.1 vector alone or encoding 25QP-GFP, 103QP-GFP, 25Q-myc, or 103Q-myc. For MDR-1 luciferase assays in SAOS-2 cells, LIPOFECTIN® transfection in 6-well plates of 2.5 µg of MDR1-luciferase plasmid (Pellegata, N. S., et al. (1995) Oncogene 11, 337-349.) and 500 ng of pcDNA3.1 vector alone or plasmids encoding p53, 20QP, 93QP, 25Q-myc, or 103Q-myc was done using pBlueScript (Stratagene, La Jolla, California) to equalize all concentrations of DNA to 5 µg.

For all luciferase assays, lysis buffer and luciferase substrates A and B buffers were used as described by the manufacturer (PharMingen, San Diego, California). Luciferase activity was measured for 10 s with a Monolight 2010 Luminometer (Analytical Luminescence Laboratory, San Diego, California). The protein concentrations of lysates were determined by Bradford analysis and luciferase activity was calculated per milligram of protein.

**Immunohistochemistry in Transgenic Mouse Brain.** The transgenic mouse line R6/2 (Mangiarini, L., et al. (1996) Cell 87, 493-506) was used [Jackson code B6CBA-TgN(HDexon 1)62) and maintained by backcrossing to (CBA X C57BL/6)F₁ mice. Genotyping and CAG repeat sizing was as described previously (Mangiarini, L., et al. (1997) Nat. Genet. 15, 197-200).

The source and working dilutions of antibodies were as follows: S830 (1:1000) was raised against an exon 1 Htt fusion protein in sheep, anti-ubiquitin (1:2000; Dako, Carpenteria, California), and anti-CBP (A22) (1:1000; Santa Cruz Biotechnology). Immunohistochemistry was performed as described previously (Davies, S., et al. (1999) Methods Enzymol. 309, 687-701) on 15-µm sections cut from isopentane frozen brains using Vectastain Elite ABC kit (Vector Laboratories, Burlingame, California). The biotinylated secondary anti-sheep antibody was from the Scottish Antibody Production Unit (Carluke, Scotland).

**Acetyltransferase Assays**. The effect of Htt proteins on the acetyltransferase activity was assayed *in vitro* by a modified technique, as described in Ail-Si-Ali, S., et al. (1998) Nucleic Acid Res. 26, 3869-3870). GST-fusion proteins were washed in 1X HAT buffer (10 mM butyrate at pH 7.5, 10% glycerol, 50 mM Tris HCl at pH 8.0, 0.5 mM DTT, 0.1 mM EDTA, 0.1 mM PMSF), then eluted with 15 mM glutathione in 1X HAT buffer containing 50 mM NaCl. Quantified by Coomasie stain, 0.6 nmol ofHtt GST fusion proteins or GST alone were incubated for 10 minutes at room temperature with 10 pmol of GST-CBP (amino acids 1,099-1,877), 4 pmol of GST-p300 (amino acids 1,195-1,707) (Upstate Biotechnology, Waltham, Massachusetts), or 360 pmol of GST-P/CAF (amino acids 87-832), as estimated by Bradford analysis, in a total volume of 55 µl.

Next, 10 µCi ¹⁴C-acetyl coenzyme A (52 mCi mmol⁻¹, DuPont NEN, Boston, Massachusetts) and 2 µg of biotinylated N-terminal H4 (amino acids 1-21) peptide (Upstate Biotechnology) were added in a volume of 4 µl, and the mixture incubated for 45 min at 30°C. A 500 µl aliquot of 1 X HAT buffer with 30 µl of a 50% slurry of streptavidin-agarose (Upstate Biotechnology) pre-equilibrated in 1X HAT buffer was then added. This mixture was rotated at 4°C for 20 min, then spun in a microfuge at 2,600 g for 2 min.

The supernatant was removed, the pellet washed twice in RIPA buffer (50 mM Tris at pH 7.5, 150 mM NaCl, 1% NP-40, 0.5% sodium deoxycholate, 0.1% SDS, 1 mM EDTA, and 0.1 mM PMSF) and counted in a liquid scintillation counter. Assays were done in quadruplicate and the standard error of the mean calculated.

**Histone acetylation analysis in PC12 cell extract.** PC12 cells, stably transfected with ecdysone-inducible (Invitrogen) F103QP-EGFP were plated, induced with 5 µM ponasterone (Invitrogen) for 48 h, and treated for the last 24 h with 5 mM sodium butyrate. Controls included uninduced and/or non-butyrate-treated cells. Cells were lysed in 1X HAT buffer with 50 mM NaCl, 0.3% NP-40, and a protease-inhibitor cocktail on ice for 10 min. A 50 µg aliquot ofwhole cell extract was analyzed by Western blot.

Separate, independently-derived, ecdysone-inducible PC12 cell lines, denoted PC12/pBWN:Htt ex1Q103-EGFP cells and PC12/pWN:Httex1Q25-EGFP (E.S. Schweitzer, UCLA), were also analyzed. These clonal cells contain a modified exon 1 of Htt inserted into an expression vector containing the *Bombyx* ecdysone-regulated element (described in Suhr, S. T., et al. (1998) Proc. Natl. Acad. Sci. U.S.A. 95, 7999-8004).

Twenty-five micrograms of whole cell extracts from PC12 cells stably transfected with plasmids encoding inducible 25QP-EGFP and 103QP-EGFP was analyzed for acetyltransferase activity. Control cells (uninduced) and cells induced with 1 µM tebufenizide for 12 h were analyzed by Western blots. Anti-acetylated histone H4, anti-histone H3 and anti-acetylated histone H3 (all from Upstate Biotechnology) were used to determine levels of acetylated histones H3 and H4 relative to levels of total H3.

***Drosophila* stocks and crosses**. Expression of polyglutamine-containing peptides is driven by the bipartite expression system activator sequence (UAS)-GAL4 in transgenic flies (Marsh, J. L. et al. (2000) Hum. Mol. Gen. 9, 13-25).

Injection of plasmids expressing Httex 1 p with 20 or 93 glutamines produced nine and ten lines, respectively. Two lines with the most severe neuronal phenotype (P463 and P465) were chosen for the experiments described herein. Polyglutamine stocks are *w*;P(w^{+mC} = UAS - Q93httexon1)^{4F1} and w;P(w^{+mC}= UAS - Q48 + myc/flag)²⁰. Constructs under the control of a yeast UAS were crossed to flies expressing the yeast GAL4 transcriptional activator (Brand, A. and Perrimon, N., *supra)* driven by the neuron-specific promoter *elav* (chromosome 2 driver for Q48 lines, Luo, L. et al. (1994) Genes Dev. 8,1787-1802, and X-chromosome driver for Htt exon 1 lines, Lin, D. M. & Goodman, C. S. (1994) Neuron 13, 507-523) that is expressed in all neurons from embryogenesis onwards: *w*;P(*w*^{+*mC*};w+; *elav-GAL4*)/*CyO*, P(*w^{+mC}=Act-GFP*)*JMR1CyO actin*-GFP or w;P(*w*^{+*mW*}.*hs = GawB)elavC155*.

The inhibitor concentration ranges tested were based on cell culture experiments (SAHA; Calbiochem, San Diego, California) or published position effect variegation studies (butyrate; Reuter, G., Dorn, R. & Hoffman, H. J. (1982) Mol. Gen. Genet. 188, 480-485). For testing the effect of *Sin3A* on polyglutamine phenotypes, we crossed *w*;P(*w^{+mC} =*UAS*-Q93httexon1*)^{4F1} virgins to *w;* P*{w*⁺*^{mC}wPw;elav-GAL4)*/*Y;Sin3A⁰⁸²⁶⁹*/*Bc Gla* males.

**Pseudopupil analysis and transgene expression**. Pseudopupil analysis allows visualization of the arrangement of rhabdomeres in the ommatidia of the compound eye (Franceschini, N. (1972) in Information processing in the visual systems of arthropods, ed. Wehner, R., pp 75-82). Adult flies were decapitated, one eye was dipped in a drop of nail polish and the head was mounted on a microscope slide. Eyes were analyzed with a Nikon EFD-3/Optiphot-2 scope using a 50x objective, and photographed with a Spot camera. At least 200 ommatidia were scored for each condition. For Western analysis of transgene expression, 20 larvae from each sample were ground in a buffer containing 0.1 M phosphate at pH7.1, 0.3 M sucrose, 0.02 mM phenylthiourea, protease cocktail and 0.1 mM PMSF, and 200 µg of total lysate were loaded per lane.

### p53 coaggregates with expanded Httexlp in inclusions in mammalian cell culture.

The amino-terminal portion ofHtt that remains after cytoplasmic cleavage and that can localize to the nucleus appears to include the polyglutamine repeat and a proline-rich region which have features in common with a variety of proteins involved in transcriptional regulation (Gerber, H.-P., et al. (1994) Science 263, 808-810). Therefore, Htt is potentially capable ofdirect interactions with transcription factors or the transcriptional apparatus and of mediating alterations in transcription.

To explore potential interactions of Htt with nuclear proteins involved in transcriptional regulation, the composition of cellular proteins associated with semipurified aggregates, or inclusions, was evaluated using a novel purification technique. Expanded polyglutamine repeat containing Httex1ps, epitope tagged at the carboxyl terminus with GFP, were expressed transiently in HEK293 cells and fluorescence analysis of fixed cells, both 103QP-GFP (complete exon 1 protein) and 103Q-GFP (a truncated form of exon 1 protein lacking the proline-rich region) formed inclusions predominantly in the cytosol, perinucleus, or media (data not shown).

The extracellular aggregates were isolated from the media and soluble proteins from the aggregate preparation (AGG) were resolved by 8% SDS-PAGE along with aliquots from the whole-cell fraction from the transfected cells (WC), using equivalent ratios of WC:AGG. The gel was Western blotted and antisera was used to examine relative levels of 103Q-GFP Htt, p53, CBP, mSin3a, mdm2, AR, NF-κB p65, and RXRα.

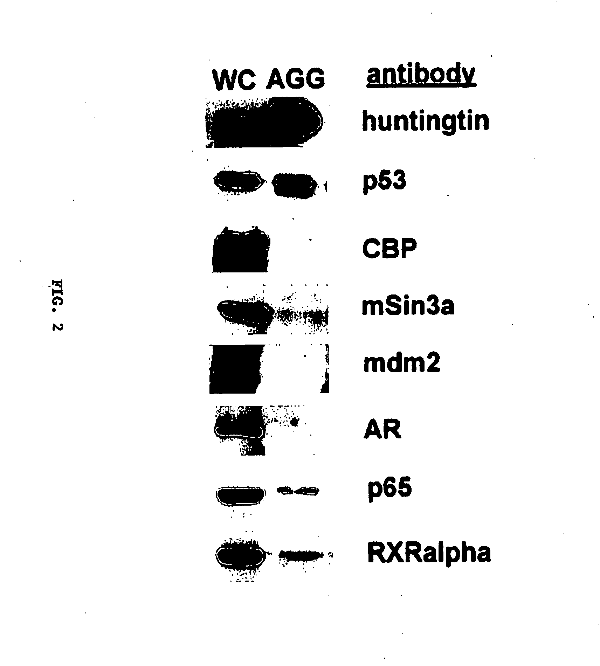

The results for aggregates generated in HEK293 cells expressing 103Q-GFP are shown in FIG. 2. The results were identical to aggregates purified from 103QP-GFP (data not shown), while no immunoreactive Htt protein was present in samples corresponding to aggregate fraction isolated from HEK293 cells expressing 25Q-GFP (data not shown).

As shown in FIG. 2, both Htt and p53 are present in 103Q-GFP-generated inclusions. Protein p53 is highly enriched compared with other cellular proteins, such as CBP and mSin3a, which have previously been shown to co-localize to inclusions by other methods. Purification of aggregates from intact cells showed a similar enrichment for p53 (data not shown). Several other nuclear proteins were analyzed for an association with the aggregates following normalization of immunoreactivity of each protein in whole-cell lysates using ¹²⁵I quantitation. The AR and mdm2 appear to be absent in the aggregate preparation, while the p65 subunit of NF-κB and RXRα appear to be present.

**Httexlp interacts with the p53 protein *in vitro* and in cell culture.** GST pull-down experiments were used to determine whether there is a direct interaction between the amino-terminal region of Htt and p53. GST fusion proteins were purified from *Escherichia coli* expressing GST-20QP (complete exon 1 containing 20 glutamines), GST-51QP (exon 1 containing 51 glutamines), GST alone, and GST-103Q (truncated exon 1 containing 103 glutamines). ³⁵S-labeled full-length p53 (amino acids 1-393) and truncated p53 (amino acids 1-347) lacking the C-terminal region were incubated with each GST fusion protein coupled to glutathione-agarose beads, the beads were washed, and similar amounts of protein were resolved by SDS-PAGE.

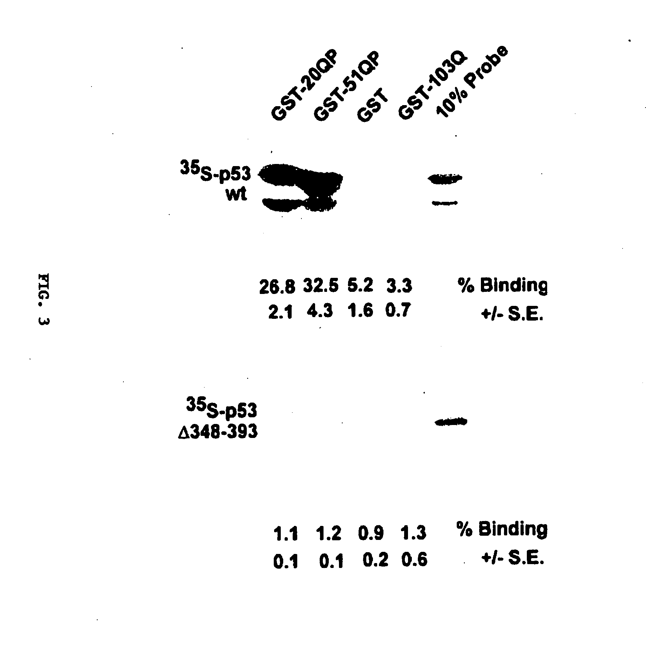

Autoradiography of a representative experiment is shown in FIG. 3. Percent binding ± SE was calculated by phosphorimager analysis and is listed below each lane. Ten percent of the input of the labeled proteins was also analyzed as shown. Slight alteration in the pattern of p53 migration in the GST-51 QP lane as compared with GST-20QP lane is due to the large amounts of GST-51QP co-migrating with labeled p53.

As shown in FIG. 3, full-length p53 binds strongly to either normal (GST-20QP) or expanded repeat (GST-51QP) protein *in vitro.* However, a long repeat containing expanded polyglutamines (GST-103Q), but lacking the proline-rich region, shows greatly diminished binding, suggesting that binding of p53 and Htt is dependent on the presence of the proline-rich region *in vitro.* Truncated p53 lacking the C-terminal amino acids 348-393 no longer interacts with Htt (Fig. 3). These data demonstrate that the amino-terminal region of Htt interacts with p53 and this interaction requires the presence of the C-terminal region of p53 as well as the proline-rich region of Htt.

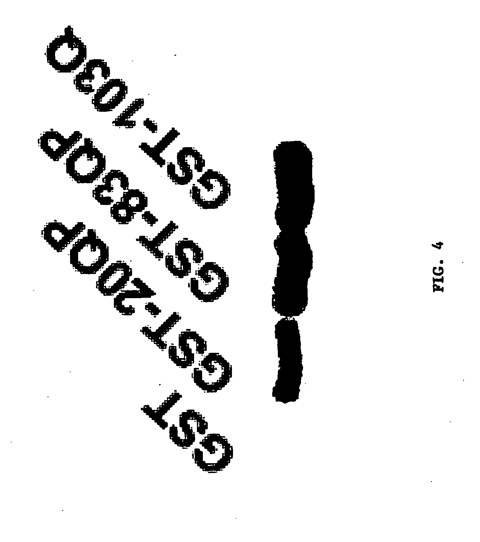

To test for an interaction between p53 and the various Httex 1ps in cell culture, additional GST pull-down experiments were performed. HEK293 cells were transiently transfected with plasmids encoding p53 and either GST, GST-20QP, GST-83QP, or GST-103Q. Cells were broken and lysates were incubated with glutathione-agarose beads. After extensive washing, beads were loaded directly on an 8% SDS gel. Western blot analysis using monoclonal anti-p53 antibody is shown in FIG. 4.

As shown, p53 was found to copurify with normal range and expanded Httex1p (GST-20QP and GST-83QP) as well as with a truncated form of an expanded Httex1p lacking the proline-rich domain (GST-103Q), demonstrating a lack of dependence on the proline-rich region of Httex1p for complex formation. This interaction of Htt and p53 was also confirmed in co-immunoprecipitation experiments from cell lysates by using anti-GFP antibody (data not shown). Co-immunoprecipitations were complicated by the fact that anti-p53 polyclonal antisera were found to directly immunoprecipitate 25QP-GFP in the absence of p53, and, similarly, polyclonal anti-Q51 antisera (Sittler, A., *supra*) could directly immunoprecipitate p53 protein in the absence of Httex1p.

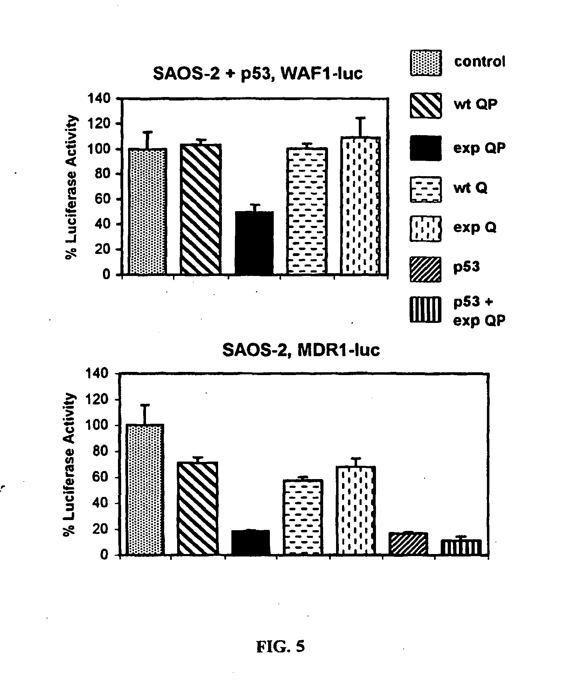

**Expanded Httex1p represses transcription.** Because of the direct interaction observed between p53 and Htt, we tested whether transient expression of Httexlp has an effect upon transcription of genes regulated by p53. The expression of a *p21*^{*WAF1*/}*^{C1P1}* reporter, WAF1-luciferase, which is transcriptionally activated by p53, was examined in SAOS-2 (p53^{-/-}) cells expressing exogenous p53, as minimal activation of this reporter occurs in the absence of p53. The results are shown graphically in FIG. 5.

Repression of WAF1-luciferase by expanded Httex1p (103QP-GFP) is observed, whereas normal range repeats (2SQP-GFP) or Htt constructs lacking the proline-rich region (2SQ-myc, 104Q-myc) produced no significant repression (FIG. 5, *upper*), indicating a role for both the expanded polyglutamine repeat and the proline-rich region in transcriptional repression.

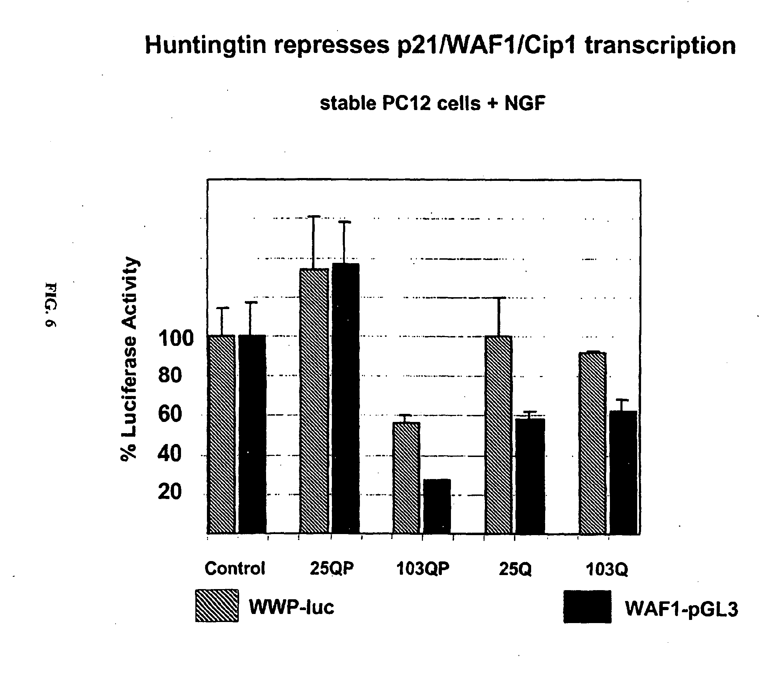

Luciferase assays were also conducted in PCl2 cells (p53^{+/+}) stably transfected with inducible Htt constructs, all containing GFP epitope tags. Upon differentiation with nerve growth factor into a neuronal phenotype, cell lines expressing 103QP-GFP repressed transcription from both the complete *p21*^{*WAF1*/}*^{C1P1}* promoter (WWP-luciferase; El-Deiry, W. S., et al. (1993) Cell 75, 817-825) and the WAF1-luciferase reporter, again in a proline- and expanded repeat-dependent manner (FIG. 6).

A promoter subject to p53-mediated transcriptional repression was also tested using luciferase assays conducted in transiently transfected SAOS-2 (p53^{*-*/*-*}) cells (Fig. 5, *lower*). The multiple drug resistance gene, *MDR-1*, has previously been shown to be transcriptionally repressed by p53 (Pellegata, N. S., *supra*; Chin, K.-V., et al. (1992) Science 255, 459-462), and this repression was shown to be dependent on the presence of the proline-rich region of p53 (Venot, C., et al. (1998) EMBO J. 17, 4668-4679). The transcription of the *MDR-1* promoter fused to luciferase was therefore examined.

SAOS-2 cells, without expression of p53, showed high levels of *MDR-1* transcription. When p53 was expressed in these cells, *MDR-1* transcription was repressed (83%). When Htt constructs were expressed in SAOS-2 cells, 93QP (Httex 1p with 93 repeats) produced extensive repression (81%) of *MDR-1* transcription, even in the absence of p53. This profile of transcriptional repression of *MDR-1* mediated by the Htt constructs in the absence of p53 was similar to that observed for the WAF1-luciferase reporter in SAOS-2 cells expressing p53 and for the *p21*^{*WAF1*/}*^{C1P1}* promoter in PC12 cells (shown graphically in FIG. 6), indicating that transcriptional repression by expanded Httex1p is dependent on the presence of the proline-rich region and on the length of the glutamine stretch.

**Expanded Httex1p interacts *in vitro* with CBP and mSin3a.** CBP is a polyglutamine-containing transcription factor which co-activates p53 transcription through interaction with p53 residues 1-73, including the activation domain and part of the proline-rich region (Gu, W., Shi, X.-L. & Roeder, R. G. (1997) Nature 387, 819-823). Since expanded Httexlp structurally resembles this region of p53 and since CBP aggregates in cell culture inclusions along with Htt (Kazantsev, A., *supra),* GST pull-down experiments were used to examine the interaction between ³⁵S-labeled CBP and various GST fusion proteins.

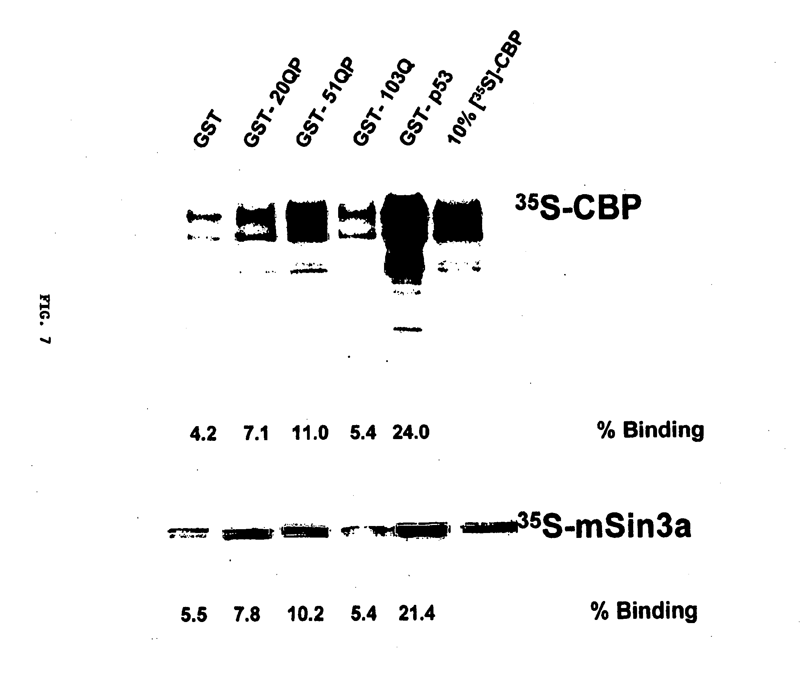

Equivalent amounts of[³⁵S]methionine-labeled CBP (I) or mSin3a (II) were mixed with GST, GST-20QP, GST-51QP, GST-103Q, or GST-p53 attached to glutathione-agarose beads, washed, and the labeled proteins bound to the beads were analyzed by SDS-PAGE. Each experiment was done in triplicate. The results are shown in FIG. 7, with the percent binding ± SE, calculated by phosphorimager analysis, listed below each lane. Ten percent of the input of the labeled proteins was also analyzed as shown.

As shown in FIG. 7-I, CBP interacts weakly with Httex 1 p in a proline- and polyglutamine repeat length-dependent manner *in vitro.*

Analogous results were obtained for mSin3a, a co-repressor that can mediate transcriptional repression through a direct interaction with p53 (Murphy, M., et al. (1999) Genes Dev. 13, 2490-2501). The mSin3a co-repressor binds the amino-terminal region of p53 through an interaction with amino acids 40-160, which includes part of the activation domain and the proline-rich region of p53. GST pull-down experiments, using the same GST fusion proteins described above, show that mSin3a also weakly interacts with Httexlp in a proline- and polyglutamine repeat length-dependent manner *in vitro* (Fig. 7-II).

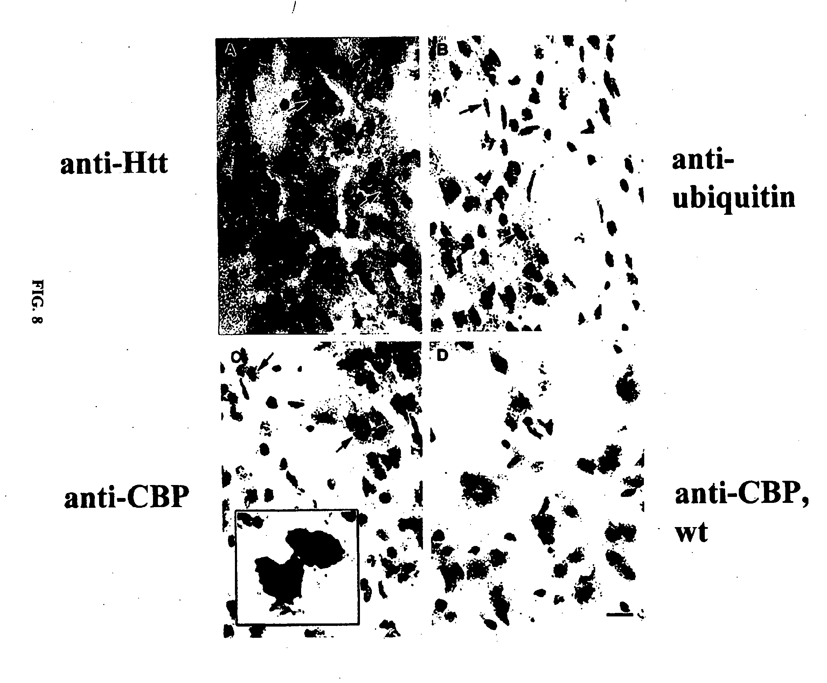

**CBP localizes to neuronal intranuclear inclusions in transgenic mice.** To demonstrate that the interactions of expanded Httex1p with CBP occur *in vivo* in the neurons of a HD transgenic mouse model, immunohistochemistry of neuronal intranuclear inclusions was performed. The results are shown in FIG. 8.

It has previously been shown that mSin3a is present *in vivo* in neuronal intranuclear inclusions in human HD patient brain sections (Boutell, J. M., *supra).* As shown in Fig. 8C, CBP localizes to nuclear inclusions against background nuclear staining in striatal brain sections isolated from 12-week-old R6/2 transgenic mice. Inclusions from these sections also stain positive for Htt (Fig. 8A) and ubiquitin (Fig. 8B) protein. CBP is also present in inclusions at 8 weeks (data not shown), suggesting that CBP localization to the aggregates is a relatively early process. The normal diffuse nuclear staining pattern of CBP is shown in wild-type littermate controls (Fig. 8D).

**The 51QP Htt fragment interacts primarily with the acetyltransferase and CH3 domains of CBP**. As shown above, truncated Htt with an expanded polyglutamine domain interacts with full-length CBP *in vitro* and co-aggregates in cell culture, suggesting two hypotheses for Htt action that are not mutually exclusive. First, it is possible that the available activity of cellular proteins containing natural polyglutamine domains could be reduced by trapping them in aggregates through polyglutamine-polyglutamine interactions. Alternatively, soluble interactions between Htt and cellular targets could directly affect enzymatic activities.

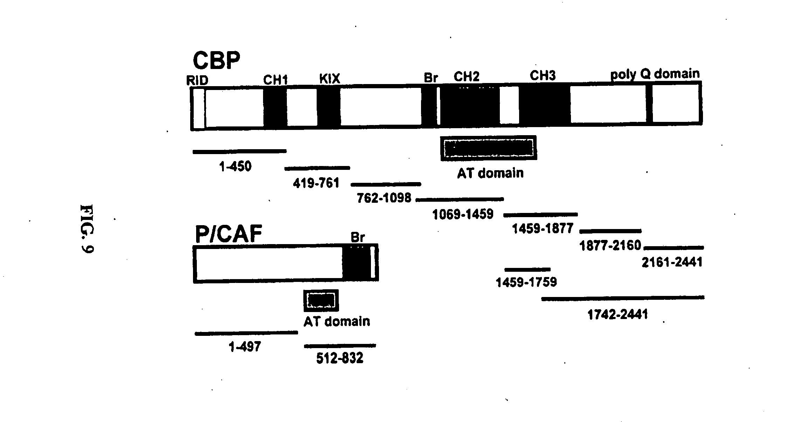

To distinguish the contributions of polyglutamine-polyglutamine interactions from those of other interactions, we investigated which functional domains of CBP are targeted for binding by Httex 1p, using GST pull-down assays. ³⁵S-labeled protein probes spanning the length of CBP (shown in FIG. 9) were incubated with glutathione-agarose beads coupled to Httex1p with 51 glutamine repeats and the proline-rich domain.

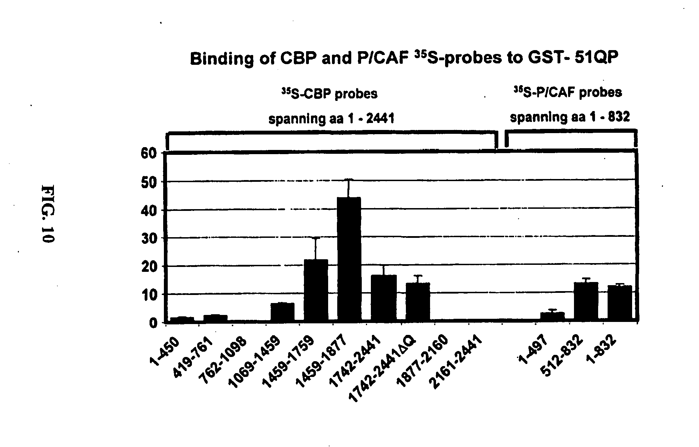

The results are shown graphically in FIG.10. Significant binding was observed to probes containing the CH2 domain and amino-terminal portion ofthe acetyltransferase domain (residues 1,069-1,459), to probes containing the carboxy-terminal portion of the acetyltransferase domain (residues 1,459-1,759), and to probes containing part of the acetyltransferase domain as well as the CH3 domain (residues 1,459-1,877).

A CBP probe containing part of the CH3 domain and the C-terminal polyglutamine stretch (residues 1,742-2,441), binds to GST-5 1QP, whereas deletion of the polyglutamine stretch (residues 1,742-2,441 ΔQ) has a negligible effect on binding. A CBP fragment containing the C-terminal polyglutamine domain of CBP (residues 2,161-2,441), did not interact *in vitro* with GST-51QP. Our data indicate that the direct interaction between the 51QP Htt fragment and CBP is not mediated by glutamine-glutamine interactions. Rather, they indicate that 51QP interacts primarily with the region of CBP containing the acetyltransferase and CH3 domains.

**Httexlp binds other proteins containing acetyltransferase domains.** We next investigated whether Httex 1p binds other proteins containing acetyltransferase domains, such as P/CAF. Unlike CBP, P/CAF does not contain a polyglutamine-rich region (see FIG. 9). Nevertheless, full-length P/CAF (residues 1-832), as well as residues containing the acetyltransferase domain (512-832), showed significant (12-13%) binding to GST-51 QP *in vitro* (FIG. 10).

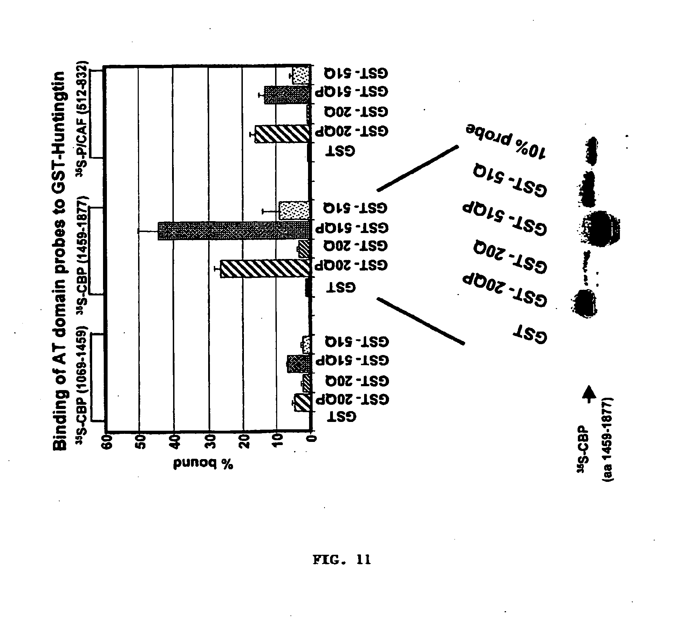

**The proline-rich region of Httexp1 enhances, but is not essential for, the *in vitro* interaction with the acetyltransferase domain of CBP**. Having found that the expanded repeat domain of Htt interacts with both CBP and P/CAF primarily through the acetyltransferase and neighboring domains, we sought to determine the features of Httexp1 that are important for binding. We tested whether the length of the polyglutamine tract or the presence of the proline-rich region affected this interaction. The results are shown in FIG. 11.

FIG. 11 is a representative autoradiogram with quantitation by phosphorimager analysis, showing binding of CBP probe 1,459-1,877 to the different GST-Htt constructs. Binding of GST-Httex 1p to the acetyltransferase/CH3 domain of CBP is enhanced when the polyglutamine repeat expands from the normal range (GST-25QP or GST-25Q) to a pathogenic range (GST-51 QP or GST-51 Q). GST fusions that contain the Htt proline-rich domain (GST-20QP and GST-51 QP) interact better with the acetyltransferase domain probes than do their counterparts without the proline-rich domain (GST-20Q and GST51Q). Thus, the proline-rich region enhances, but is not essential for, the *in vitro* interaction.

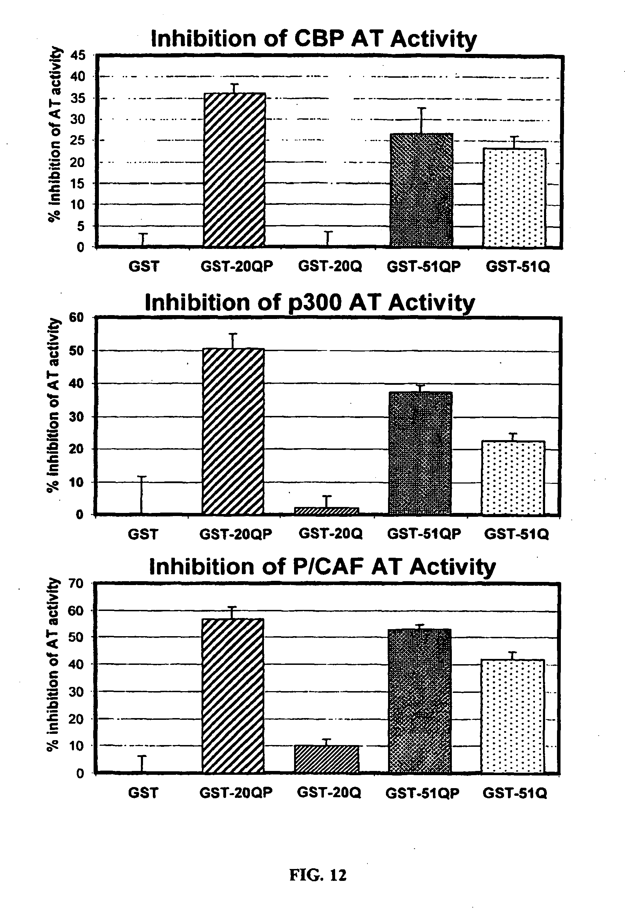

**Httex1p inhibits acetyltransferase activity *in vitro.*** Httex1p interacts *in vitro* with the acetyltransferase domains of P/CAF and CBP. Accordingly, the effect of Httex1p on the acetyltransferase activity of widely-expressed transcriptional co-activator proteins CBP, p300, and P/CAF was measured *in vitro.* Equimolar amounts of GST, GST-20QP, GST-20Q, GST-51 QP, and GST-51Q proteins were incubated with GST-CBP-AT (residues 1,099-1,877), GST-p300-AT (residues 1,195-1,707), or GST-P/CAF-AT (residues 87-832), containing the acetyltransferase domains. Following a short incubation, the acetylation of an H4 peptide was monitored. The presence of the Htt GST-fusion protein, GST-51Q, as well as GST-20QP and GST-51QP, which contained the proline-rich region, significantly reduced acetyltransferase activity of CBP, p300, and P/CAF, whereas GST and GST-20Q did not, as is shown graphically is FIG. 12.

This demonstrates that the direct interaction of Htt with acetyltransferase domains (FIG. 11) inhibits acetyltransferase function and that long polyglutamine stretches are more potent than short normal-range repeats. The presence of the proline-rich region increases the inhibitory potential of polyglutamine polypeptides. Surprisingly, GST-51Q inhibits acetyltransferase activity almost as well as GST-51 QP, demonstrating that expanded polyglutamine stretches can interact with acetyltransferase domains and inhibit function without the proline-rich region.

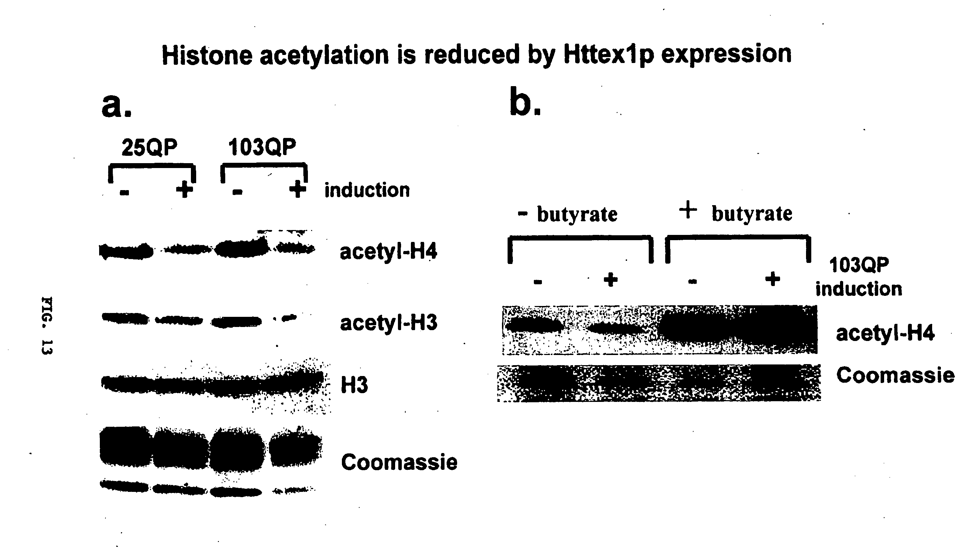

**Httex1p reduces the acetylation of histones H3 and H4 when expressed in cell culture.** Because Httex1p interacts directly with acetyltransferase domains and inhibits their function *in vitro,* acetylation levels ofH3 and H4 in PC12 cells stably transfected with inducible Httex1p constructs were analyzed. FIG. 13 is a Western blot showing levels of acetyl-H3 and acetyl-H4 with and without induction of Httex1p constructs. As shown in FIG. 13a, expression of Httex1p with a normal range repeat (25QP) reduces acetylation of both histones H3 and H4 compared to uninduced cells, with expression of an expanded repeat Httex1p (103QP) showing an even greater reduction of acetylation.

To verify these results, acetylation levels in a second transformed cell line, generated in a different strain of PC12 cells, were analyzed. Induction of an expanded polyglutamine repeat Httex1p (103QP) in the second PC12 line also leads to reduction of histone H4 acetylation in whole cell extracts compared to uninduced cells, shown in FIG. 13b. When these cells are treated with the HDAC inhibitor sodium butyrate, levels ofH4 acetylation in both uninduced and induced cells are increased (FIG. 13b). Trichostatin A and suberoylanilide hydroxamic acid (SAHA) also increased acetylation of H3 and H4, reversing the decrease induced by Httex1p (data not shown). While acetylation levels are altered, total histone levels in whole cell extracts, determined by Coomassie blue staining of total cell lysates, are unchanged by induction of Htt proteins. Thus, expression ofHttex 1 p causes a global reduction in acetylation of histones H3 and H4 that is reversed in the presence of HDAC inhibitors.

As shown in FIGs. 11 and 12, both normal-repeat Htt and expanded Htt bind to and inhibit acetyltransferase activity. This raises the question of why pathology is only associated with expanded repeat Htt. It has been shown that expanded Htt can be proteolytically processed and that the pathogenic fragment resulting from proteolytic processing localizes to the nucleus where it is capable of inhibiting acetyltransferase activity (Paulson, H. L. (1999) Am J Hum Genet 64, 339-345; Klement, I. A. et al. (1998) Cell 95, 41-53; Saudou, F., et al. (1998) Cell 95, 55-66). However, unexpanded normal-repeat Htt does not normally localize to the nucleus (Sapp, E. et al. (1997) Ann. Neurol. 42, 604-611) and therefore is not present in the appropriate cellular compartment to inhibit nuclear CBP activity. Indeed, unexpanded polyglutamine repeats can cause pathology. For instance, unexpanded human ataxin-1 protein, which contains 30 glutamines, is normally localized in the nucleus; if expressed at sufficiently high levels there, it can produce neurodegenerative phenotypes similar to expanded 82-glutamine ataxin-1 in either *Drosophila* or mice. (Fernandez-Funez, P. et al. (2000) Nature 408, 101-106.) Thus, polyglutamine pathogenesis depends heavily on both level and location.

**Histone deacetylase inhibitors rescue progressive neuronal degeneration and lethality in *Drosophila*.** The reduced acetylation of histones observed in the presence of expanded repeat Httex1p *in vitro* (with or without the proline-rich tract) and the subsequent reversal of this effect with HDAC inhibitors in cell culture, suggested that reduced acetyltransferase activity may be an important component of polyglutamine pathogenesis *in vivo.* Expanded polyglutamine peptides alone (Marsh et al., *supra*) as well as expanded repeat Httex 1p, as shown herein, are intrinsically cytotoxic and cause reduced viability and neuronal degeneration when expressed in *Drosophila* neurons.

If polyglutamine pathology involves suppression of protein acetylation, then one would predict that inhibition of the deacetylation process by two completely independent mechanisms (for example, pharmacologically or genetically) would slow or reduce polyglutamine pathogenesis *in vivo.* To test this hypothesis, transgenic flies were engineered to express either Httex1p or just polyglutamine peptides in neurons. The effects of feeding flies with the HDAC inhibitors butyrate and SAHA, and of genetically reducing fly HDAC activity, on both lethality and degeneration of photoreceptor neurons, was evaluated.

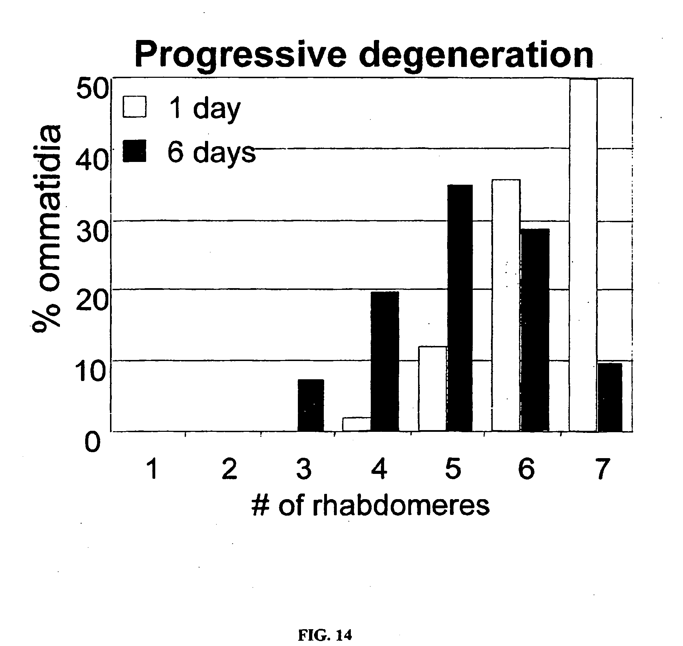

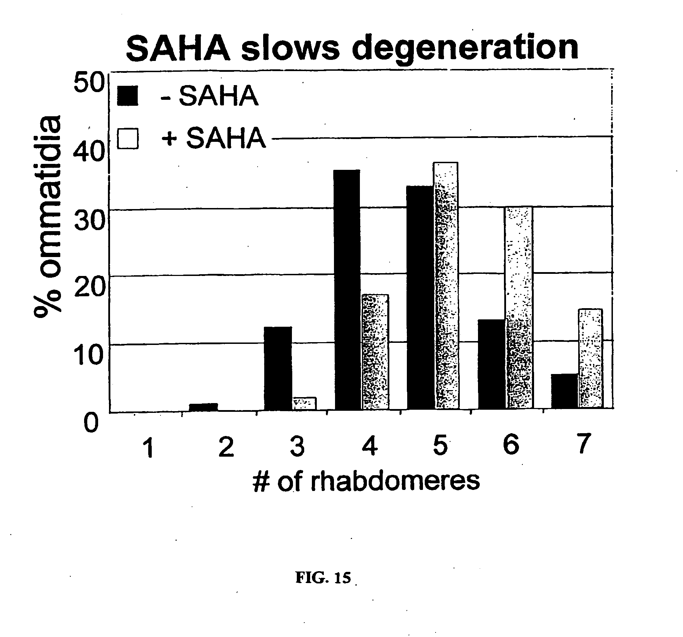

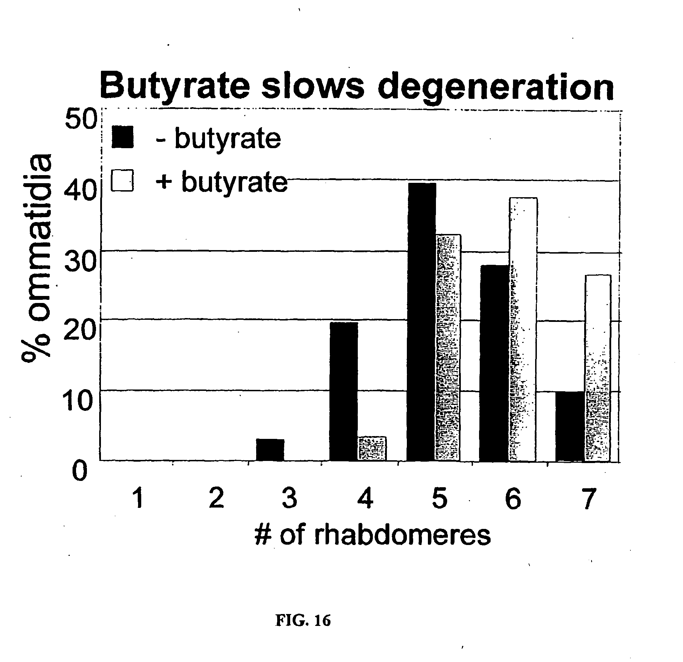

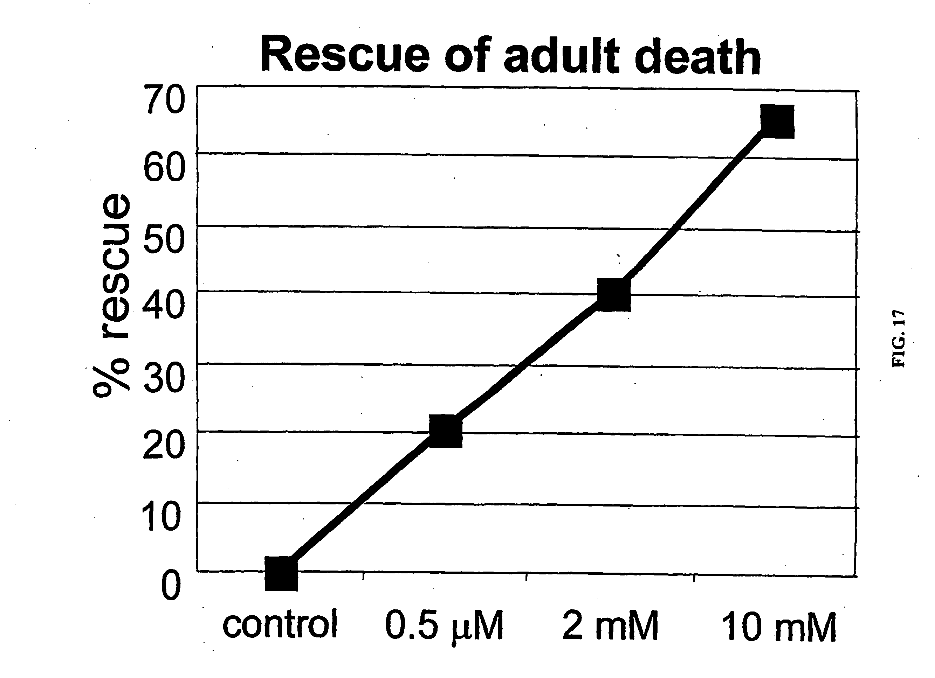

Neurodegeneration is most readily observed in the fly compound eye, composed of a regular trapezoidal arrangement of seven visible rhabdomeres (subcellular light gathering structures) produced by the photoreceptor neurons of each *Drosophila* ommatidium. As shown graphically in FIG. 14, expression of Httex1p with an expanded tract of 93 glutamines (Q93) leads to a progressive loss of rhabdomeres. Rather than the normal 7 visible rhabdomeres, the number of rhabdomeres seen in flies expressing Httex1p Q93 progressively declines from an average of 6.35 at day 1, to 5.13 and 4.66 at days 6 and 12 post-eclosion respectively (i.e. following emergence from the pupal case as an adult). Rearing larvae expressing Httex1p Q93 on either SAHA- or butyrate-containing food reduces the level of degeneration observed, as shown in FIGs. 15 and 16, respectively. Expression of the Httex1p Q93 transgene also results in approximately 70% lethality (data not shown), and early adult death (FIG. 17). In contrast, animals fed the HDAC inhibitor SAHA show increased viability (10 µM SAHA suppresses lethality to 45%; data not shown), and early adult death is markedly repressed in a concentration-dependent manner (FIG. 17).

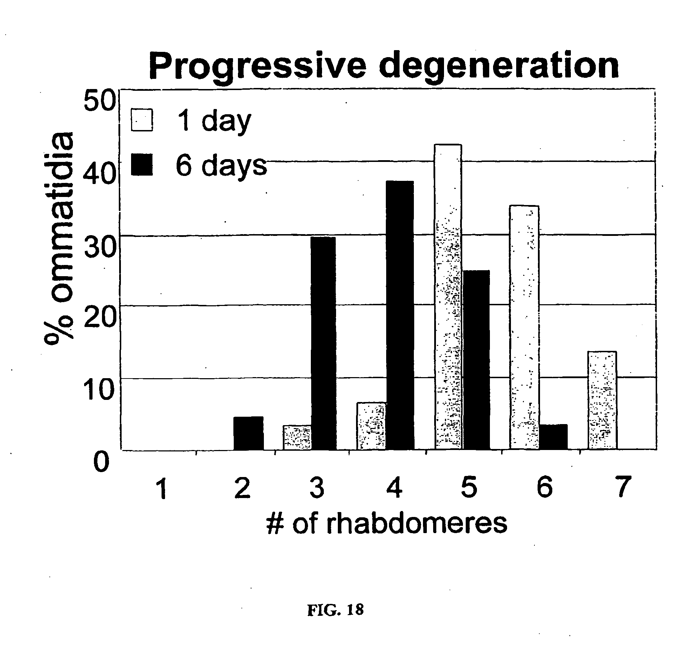

The effects of HDAC inhibitors on transgenic flies expressing extended polyglutamine peptides alone (Q48) were similar to those described above: Q48 flies fed butyrate or SAHA have the same distribution of rhadomeres by day 6 as 1-day-old flies (data not shown), whereas their siblings that did not receive HDAC inhibitors showed a significant degeneration of rhabdomere number over time (average of 5.47 at day 1 versus 3.92 at day 6, as shown in FIG. 18).

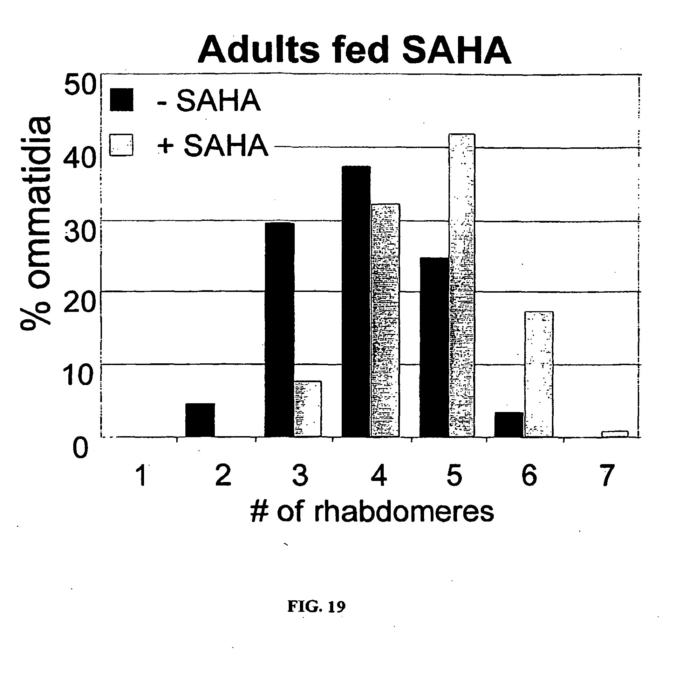

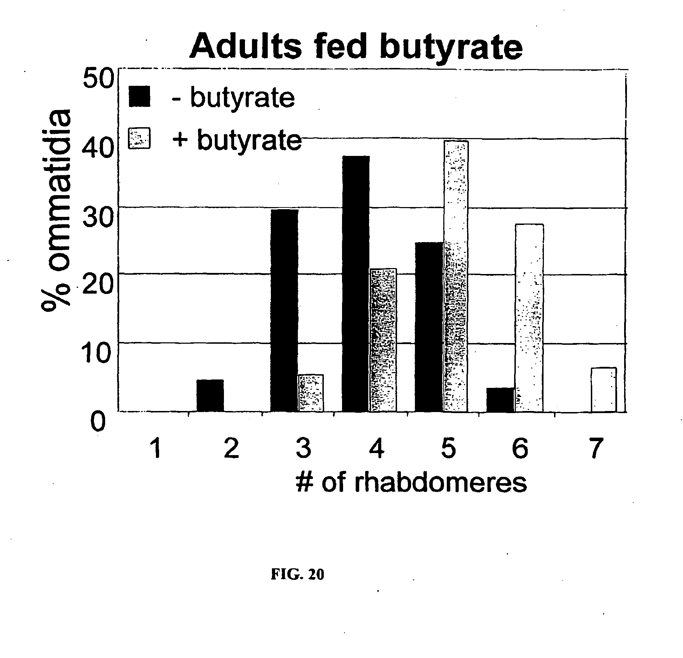

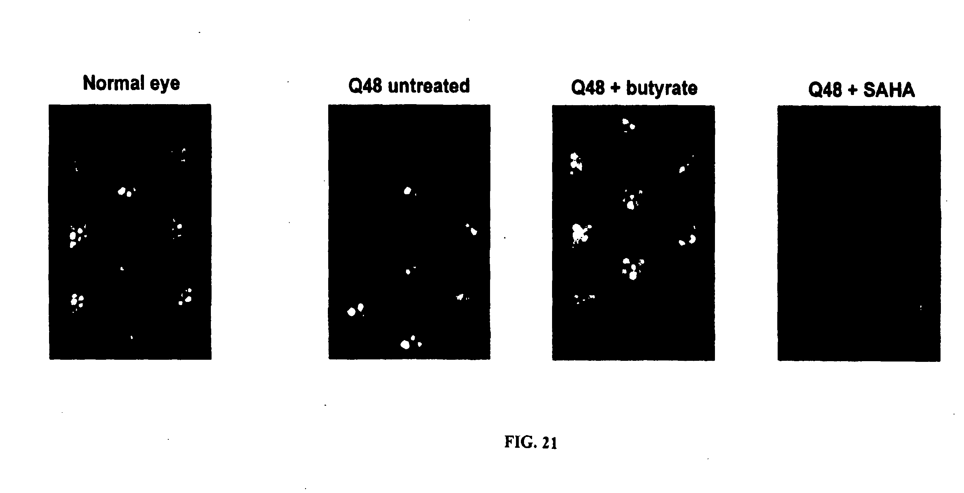

Even when fed HDAC inhibitors only after emerging from the pupal case as adults, progressive degeneration of photoreceptor neurons was still prevented (FIG. 19, showing the effect of SAHA administration, and FIG. 20, showing the effect of butyrate administration). Photographs of ommatidia from Q48-expressing flies with and without HDAC inhibitors are shown in FIG. 21. As shown, administration of either butyrate or SAHA disrupts the neurodegenerative effect of Q48. Therefore, even when administered to animals already exhibiting neurodegeneration, HDAC inhibitors markedly retard (or arrest) further neuronal degeneration. Thus, HDAC inhibitors rescue pathological effects ofboth polyglutamine peptides and Htt exon 1 polypeptides *in vivo.*

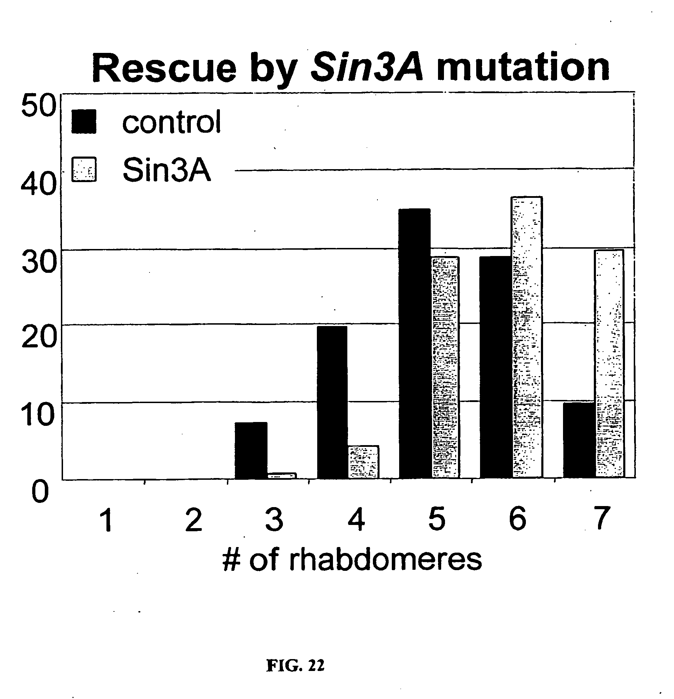

It is possible that HDAC inhibitors might affect cellular processes other than the deacetylase pathways. As an independent test of the significance of acetylation levels in the pathogenic process, acetylation levels were genetically manipulated and the resulting pathology examined. The *Drosophila Sin3A* locus encodes a co-repressor protein that is a component of HDAC complexes (Neufeld, T. P., Tang, A. H. & Rubin, G. M. (1998) Genetics 148, 277-286). Reducing the levels of HDAC by a partial loss of function mutant, S*in3A*⁰⁸²⁶⁹, in heterozygotes increased the viability of Httex1p Q93 flies from 29% to 65% and led to a reduction in the extent and rate of neurodegeneration, as shown graphically in FIG. 22. Because the mutant allele represents a partial loss of Sin3A function, the effect upon rescue of neurodegeneration may be less than that observed in the presence of HDAC inhibitors. Thus both genetic and pharmacological reductions in the activity ofHDAC reduce the rate and extent of polyglutamine-induced pathology.

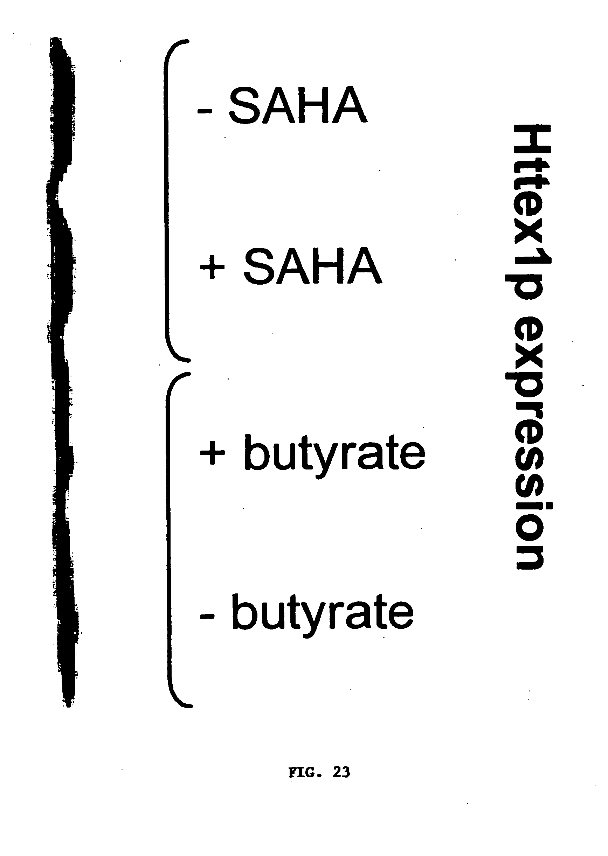

To rule out the possibility that the rescue of degeneration and lethality by HDAC inhibitors was simply due to altered expression of the polyglutamine transgenes in the presence of HDAC inhibitors, extracts from larvae expressing Httex 1 pQ93 and treated either with solvent alone or with SAHA or butyrate were prepared. Similar amounts of protein, as determined by Bradford assays and confirmed by Coomassie staining, were resolved on an SDS-PAGE gel and Western blotted. The Western blot, probed with anti-Htt antibody, is shown in FIG. 23 and demonstrates that transgene expression was unaltered by the presence of HDAC inhibitors.

Although we have shown functional interactions of Htt with CBP, P/CAF and p300, these results do not exclude the possibility that other acetyltransferases may be targeted, but they do suggest that treatments that raise global level of acetylation may be effective in ameliorating the effects of Huntington's disease and other neurodegenerative processes, even after the onset of symptoms.

**Several HDAC classes are able to suppress the polyglutamine phenotypes.** The inhibitor SAHA inactivates Class I and Class II histone deacetylases but doesn't affect the activity of Class III HDAC proteins. Sin3A mutants decrease the amount of only one HDAC complex, the Sin3A complex.

Here, we show that genetically disrupting several different HDAC complexes and reducing the dosage of Class III histone deacetylases also improves the polyQ phenotype. But we also find that the phenotype is insensitive to changes in the level of some gene products.

The Sir2 gene encodes a Class III histone deacetylase that is not affected by classical HDAC inhibitor compounds. To determine the effect of reduced Sir2 level on polyQ pathogenesis, we crossed elav>GAL4; Httex 1pQ93/TM6 males to Sir2⁰⁵³²⁷/CyO females, scored the eclosing progeny and investigated neuronal cell loss using the pseudopupil technique described above.

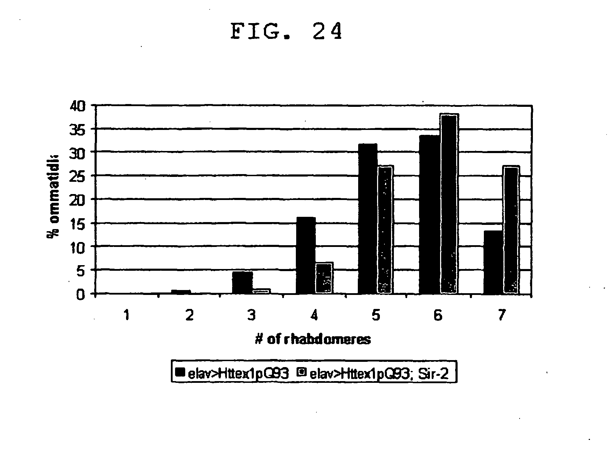

The Sir2 mutation reduced both lethality and neuronal cell loss. Since only 29% of the polyQ expressing animals survive at 25°C, 57% of the Sir2⁰⁵³²⁷ heterozygous polyQ expressing flies survived till adulthood. The average number of visible rhabdomeres in one ommatidia of the eye was 5.84 compared to 5.33 in the control group, which carried wild type Sir2 alleles, as shown in FIG. 24.

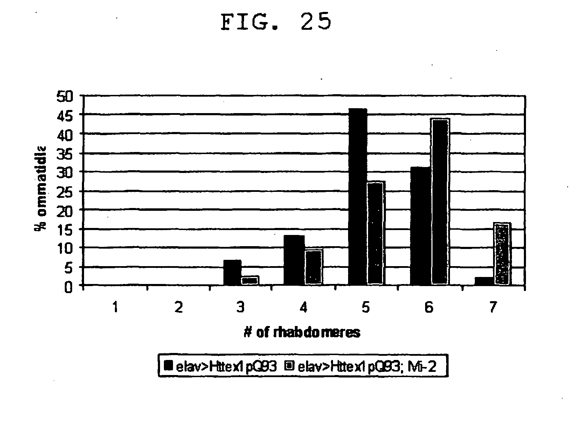

The Mi-2 gene encodes a HDAC Class I co-factor which is a critical subunit of the NuRD HDAC complex. To determine whether the disruption of this complex can suppress the polyQ phenotypes, we crossed elav>GAL4; Httex1pQ93/TM6 males to Mi-2^{j3D4}/TM3 females, scored the eclosing progeny and investigated neuronal cell loss using the pseudopupil technique. PolyQ expressing flies carrying the Mi-2^{j3D4} mutant allele show 53% viability compared to the 29% viability ofthe polyQ expressing flies with wild type Mi-2 alleles. The average number of visible rhabdomeres in one ommatidia of the eye is 5.63 compared to 5.09 in the control group, shown in FIG. 25.

Studies with other genetic elements have shown that multiple, but not all, HAT/HDAC complexes may be effective targets for reducing polyglutamine mediated neurodegeneration. For example, we have not yet found a demonstrably significant effect of reducing the genetic elements Su(var)205, CtBP, and Pcaf, while Sir2, Mi-2 and Df(HAT) do exhibit measurable responses. These results indicate that multiple, but not all, HAT/HDAC complexes can be effective targets for reducing polyQ mediated neurodegeneration.

These data suggest that combinatorial drug treatment strategies may be effective and that target identification will allow one to focus on the subset of loci that are most relevant. Small molecule inhibitors of Sir2 have recently been described and include sirtinol and vitamin B3 (Luo, et al. (2001) Cell 107: 137-148) and splitomicin (Bedalov, et al. (2001) Proc. Natl. Acad. Sci. 98, 15113-15118).

**HDAC inhibitors as therapeutic agents.** As shown herein, Htt peptides can lead to reduced levels of acetylation and transcription, both by binding to acetyltransferase domains and inhibiting soluble activity, and by sequestering polyglutamine-containing transcription factors (such as CBP and others) by trapping them into aggregates. When tested *in vivo* in *Drosophila* models of polyglutamine pathogenesis, inhibition of the deacetylation process by two independent mechanisms - pharmacological (HDAC inhibitors) and genetic (reduction ofSin3A activity) - reduced degeneration of photoreceptor neurons and lethality.

These results demonstrate a role for the state of acetylation in the pathogenic process and, more specifically, the usefulness of deacetylase inhibitors as therapeutic agents for Huntington's disease and other related diseases. Several HDAC inhibitors, including SAHA (Marks, P. A., Richon, V. M. & Rifkind, R. A. (2000) J. Natl. Cancer Inst. 92, 1210-1216), are currently approved by the U.S. Food and Drug Administration (FDA) for use in other clinical settings or are in phase I clinical trials, particularly for the treatment of cancer.

The present invention extends the usefulness of these inhibitors to the treatment of Huntington's disease and other neurodegenerative diseases, such as those caused by polyglutamine peptides (Kennedy's disease, dentatorubral-pallidoluysian atrophy, spinocerebellar ataxia, types 1, 2, 3 (Machado-Joseph), 6 and 7, and TBP (severe cerebellar atrophy)) or characterized by protein aggregation in the brain (Alzheimer's disease, Parkinson's disease, amyotrophic lateral sclerosis, Pick's disease, prion disease and other spongiform encephalopathies). Acetylase inhibitors are also useful in the treatment of psychiatric diseases such as schizophrenia, bipolar disorder, and depressive illness, and in the treatment of epilepsy.

**Polyglutamine repeat diseases**. As shown herein, flies expressing polyglutamine peptides, even in the absence of specific disease polypeptides, exhibit progressive neurodegeneration, an effect that can be suppressed by either pharmacologic inhibition (using HDAC inhibitors) or genetic inhibition (reduced function Sin3A). See FIGS. 18-22. Reduced histone acetylation has also been demonstrated in yeast expressing polyglutamine peptides (Hughes, et al. (2001) Proc. Natl. Acad. Sci. 98, 13201-06). This suggests that reduced acetylation is a common mechanism of all polyglutamine repeat diseases and, thus, that such diseases can be treated with deacetylase inhibitors.

With respect to Huntington's disease, using *in vitro,* cell culture and *Drosophila* assays, the present inventors have shown that acetylation of his tones (and, presumably, other cellular proteins) is altered by Httexlp with expanded polyglutamines; moreover, the use of histone deacetylase inhibitors suppresses the pathogenic phenotype in *Drosophila* expressing Httex1p with expanded polyglutamines. The present inventors have also shown that Htt binds to the acetyltransferase domain of CBP and P/CAF, and inhibits histone acetylation by the acetyltransferase domains of CBP, P/CAF, and p300 *in vitro.*

Similarly, with respect to Kennedy's disease (spinobulbar muscular atrophy), histone acetylation is decreased in cell culture in the presence of expanded polyglutamine androgen receptor, and histone deacetylase inhibitors suppress this decrease as well as expanded polyglutamine androgen receptor mediated cell death in culture (McCampbell, A. et al. (2001) Proc. Natl. Acad. Sci. 98, 15179-84). In addition, the present inventors have shown that expanded polyglutamine androgen receptor binds more efficiently to P/CAF than does normal unexpanded androgen receptor (data not shown).

Also, atrophin, both expanded and normal, binds to the AT and CH3 domains of CBP and to P/CAF, and expanded polyglutamine Ataxin-1 binds P/CAF better than normal unexpanded Ataxin-1 (data not shown).

All of the above supports a role for polyglutamine expansions in affecting protein acetylation and, significantly, the potential for deacetylase inhibitors as therapeutic agents in treating polyglutamine repeat diseases such as Huntington's disease, Kennedy's disease, dentatorubral-pallidoluysian atrophy, spinocerebellar ataxia, types 1, 2, 3 (Machado-Joseph), 6 and 7, TBP (severe cerebellar atrophy), and others.

**Aggregation diseases**. A common feature of many neurodegenerative diseases is the presence of protein aggregation in the brain. Examples of neurodegenerative diseases characterized by protein aggregation include Alzheimer's disease, Parkinson's disease, amyotrophic lateral sclerosis, Pick's disease, prion disease and other spongiform encephalopathies.

The present inventors have demonstrated that expanded polyglutamine Httexp1 co-aggregates with p53 in inclusions generated in cell culture, and interacts *in vitro* with CBP and mSin3A; moreover, CBP localizes to neuronal nuclear inclusions in a transgenic mouse model of Huntington's disease. See FIGs. 2-4, 7 and 8. As discussed above, such protein aggregations reduce acetylation levels in cells by sequestering cellular proteins having acetyltransferase activity, such as the transcription factor CBP, leading to neurodegeneration and cell death. The present inventors have demonstrated that such reduced acetylation levels are compensated by the addition of deacetylase inhibitors, suppressing the pathogenic phenotype.

This indicates that neurodegenerative diseases characterized by protein aggregation also involve sequestration of acetyltransferases and, as a result, altered acetylation levels. For example, amyloid precursor protein, a protein implicated in nerve cell damage in Alzheimer's disease, has been shown to interact with histone acetyltransferase (Cao, X. and Sudhof, T.C. (2001) Science 293, 115-120), which, in view of the present inventors' findings, suggests a role for altered acetylation levels in the Alzheimer's disease state. Accordingly, deacetylase inhibitors such as SAHA and butyrate have therapeutic potential for the treatment of aggregation diseases such as Alzheimer's disease, Parkinson's disease, amyotrophic lateral sclerosis, Pick's disease, prion disease and other spongiform encephalopathies.

**Psychiatric Diseases**. Finally, altered acetylation levels are linked to a number of psychiatric diseases. For example, the present inventors have demonstrated that KSCa3, a polyglutamine repeat-containing potassium channel gene and a candidate gene for schizophrenia, binds to the acetyltransferase and CH3 domains of CBP and to P/CAF (data not shown). Also, it has recently been shown that there is transcriptional repression of two genes, reelin and GAD67, both of which may use p300/CBP as co-activators, in the brains of schizophrenia and bipolar disorder patients. Guidotti, A. et al. (2000) Arch. Gen. Psychiatry 57, 1061-69.

In addition, valproate, a drug used to treat bipolar disorder, depressive illness and seizure disorders such as epilepsy, has recently been shown to have histone deacetylase activity. Gottlicher, M. et al. (2001) EMBO J. 20, 6969-6978; Phiel, C.J. et al. (2001) J. Biol. Chem. 276, 36734-36741.

Further, strong clinical evidence exists to support the anticonvulsant efficacy of a ketogenic diet in the treatment of epilepsy. A ketogenic diet increases the levels of a substance with structural similarity to the histone deacetylase inhibitor butyrate (beta-hydroxy butyrate) which may possess histone deacetylase inhibitor function. (Rho, J.M., et al. (1999) Epilepsy Research 37, 233-240.) Therefore, a ketogenic diet should also prove useful in the treatment of neurodegenerative diseases by increasing endogenous histone deacetylase inhibitors.

All of the above demonstrates the therapeutic usefulness of deacetylase inhibitors in the treatment of psychiatric disorders such as schizophrenia, bipolar disorder, depressive illness and seizure disorders such as epilepsy.

**Histone deacetylase inhibitors.** Known histone deacetylase inhibitors include butyrates (including sodium butyrate and sodium phenylbutyrate), tributytrin, trichostatin A (TSA), TPX-HA analog (CHAP compounds built from TSA and cyclic tripeptides, hydroxamic acid based), trapoxin, MS-275 (MS-27-275), NSC-706995, NSC-625748, NSC-656243, NSC-144168, psammaplin analogues, oxamflatin, apicidin and derivatives, chlamydocin analogues, dimethyl sulfoxide, depudecin, scriptaid, isoquinoline imide, depsipeptide (FR901228), N-acetyl dinaline, SAHA, suberic bis-hydroxamic acid, pyroxamide and analogues, m-carboxy cinnamic acid bis-hydroxamic acid (CBHA), cotara 1311-chTNT-1/B, CI-944, valporate, splitomicin, sirtinol, vitamin B3, allyl sulfur compounds, and dimethylaminobenzamidylcaprylic hydroxamate (DBCH).

The histone deacetylase inhibitors fall into six structural classes, as shown in Table 2. See Kramer, O.H. et al. (2001) Trends in Endo. & Metab. 12, 294-300.

**Table 2**

| **Structural Class** | **Examples of HDAC Inhibitors** |
|---|---|
| Short chain fatty acids and derivatives | Butyric acid Phenylbutyrate |
| Epoxides | Depudecin |
| Cyclic tripeptides | Depsipeptide |
| Cyclic tripeptides with a 2-amino-8-oxo-9,10-epoxy-decanoyl moiety | Trapoxin |
| Hydroxamic acids | TSA, SAHA, Scriptaid, Oxamflatin |
| Benzamides | MS-27-275 |

The chemical structures of these HDAC inhibitors are shown in Table 3.

**TABLE 3**

| **HDAC Inhibitor** | **Chemical Structure** |
|---|---|
| Butyric acid | 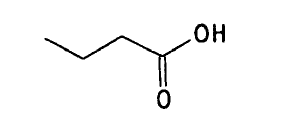 |
| Phenylbutyrate | 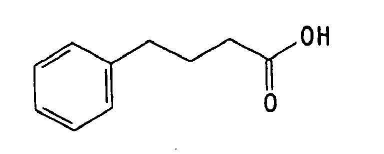 |
| Depudecin | 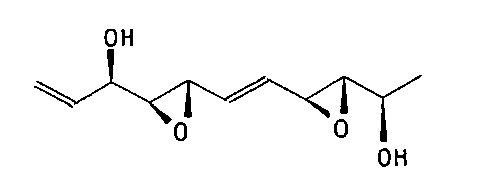 |
| Trapoxin | 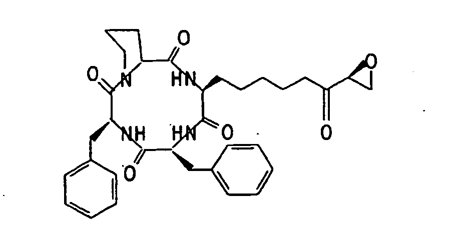 |
| Depsipeptide | 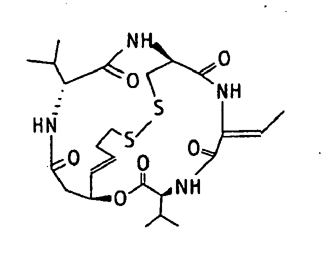 |
| TSA | 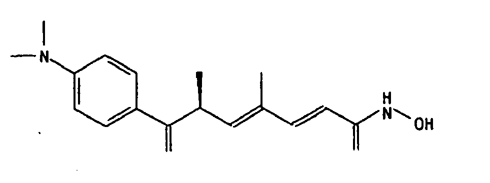 |
| SAHA | 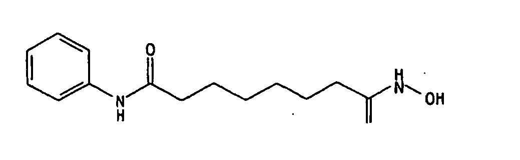 |
| Scriptaid | 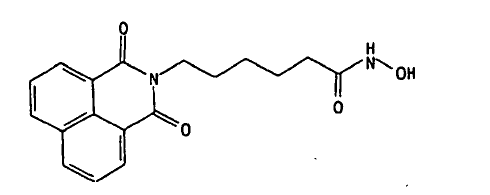 |
| Oxamflatin | 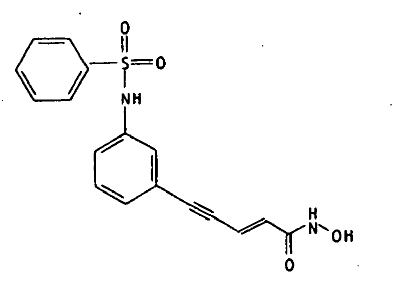 |
| MS-275 | 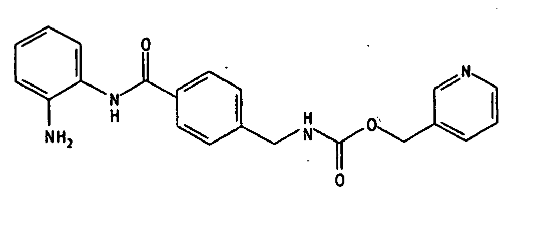 |

General formula I below represents some of the deacetylase inhibitors useful in the practice of the present invention:

[Q---T---M---B---R---S]ₙ (I)

Wherein,
S is selected from H, saturated or unsaturated, straight or branched, chiral or achiral, cyclic or acyclic hydrocarbyl group with 1 to 10 carbons, -OR, and - NR₁, where R, is selected from H, saturated or unsaturated, straight or branched, chiral or achiral, cyclic or acyclic hydrocarbyl group with 1 to 10 carbons, aryl, aralkyl, heterocyclyl, and heterocyclylalkyl, optionally substituted with from 1 to 3 substituents selected from halogen, amino, alkylamino, dialkylamino, pyrrolidino, piperidino, acylamino, cyano, aminomethyl, hydroxy, alkoxy, carboxyl, alkoxycarbonyl and nitro;
R is selected from either -CO-X- or -X-CO-, where X is selected from N-R₂ or is absent and R₂ is selected from H, saturated or unsaturated, straight or branched, chiral or achiral, cyclic or acyclic hydrocarbyl group with 1 to 10 carbons, aryl, acyl and aralkyl, heterocyclylalkyl such as 2,3 and 4-pyridylmethyl;
R₁ and R₂ may combine to form a heterocyclic ring;
B is selected from aryl, saturated or unsaturated, straight or branched, chiral or achiral, cyclic or acyclic hydrocarbyl group with 1 to 10 carbons, heterocyclyl or absent;
M is selected from saturated or unsaturated, straight or branched, chiral or achiral, cyclic or acyclic hydrocarbyl group with 1 to 10 carbons or aryl;
T is selected from urethane group (-O-CO-NH- or-NH-CO-O-), amide group (-NH-CO-or-CO-NH-), sulfonamide group (-SO₂-NH-or -NH-SO₂-), urea group (-NR₁-CO-NR₂-), where R1 and R2 are as defined before, imide group (R3-CO-N-CO-R4), where R3 and R4 may combine to form an aryl group such as 1,8-naphthyl moiety or carbonyl group (-CO-) or absent;
Q is selected from H, OH, saturated or unsaturated, straight or branched, chiral or achiral, cyclic or acyclic hydrocarbyl group with 1 to 10 carbons or aryl, substituted aryl, aralkyl, substituted aralkyl, heterocyclyl, substituted heterocyclyl, heterocyclylalkyl and substituted heterocyclylalkyl, where the substituents, from 1 to3, are selected from halogen, amino, alkylamino, dialkylamino, pyrrolidino, piperidino, acylamino, cyano, aminomethyl, hydroxy, alkoxy, carboxyl, alkoxycarbonyl, nitro or absent; and n is 1 or 2.

Several deacetylase inhibitors have been previously synthesized and used for other purposes. See, e.g., Kramer, *supra,* and the references cited therein; Uesato, S. et al. (2002) Bioorg. & Med. Chem. Lett. 12, 1347-1349; Finnin, M.S. et al. (1999) Nature 401, 188-193; Richon, V. M. et al. (2000) Proc. Natl. Acad. Sci. 97, 10014-10019; Richon, V. M. et al. (1998) Proc. Natl. Acad. Sci. 95, 3003-3007; Marks, P. A. et al. (2001) Curr. Opin. Oncol. 13, 477-483; U.S. Pat. No. 6,087,367; U.S. Pat. No. 5,773,474; U.S. Pat. No. 5,840,960; U.S. Pat. No. 5,932,616; U.S. Pat. No. 5,700,811; U.S. Pat. No. 5,668,179; U.S. Pat. No. 5,608,108; U.S. Pat. No. 5,369,108; U.S. Pat. No. 5,330,744; and U.S. Pat. No. 5,175,191.

It is possible to modify the deacetylase inhibitors described herein to produce analogues which have enhanced characteristics, such as greater specificity for the enzyme, enhanced solubility or stability, enhanced cellular uptake, or better binding activity. Salts of products may be exchanged to other salts using standard protocols.

Other deacetylase inhibitors, as well as modified deacetylase inhibitors, suitable for practice of the present invention will be apparent to the skilled practitioner, and include any compound that inhibits the deacetylase activity, even if not structurally similar to the compounds shown above.

The modes of administration for the deacetylase inhibitors include, but are not limited to, oral, transdermal, transmucosal (e.g., sublingual, nasal, vaginal or rectal) or parenteral (e.g., subcutaneous, intramuscular, intravenous, bolus or continuous infusion). The actual amount of drug needed will depend on factors such as the size, age and severity of disease in the afflicted individual.

The actual amount of drug needed will also depend on the effective inhibitory concentration ranges ofthe various deacetylase inhibitors. Different deacetylase inhibitors have different effective inhibitory concentration ranges, as shown in Table 4.

**Table 4**

| **Effective Inhibitory Concentration Range** | **Deacetylase Inhibitor(s)** |
|---|---|
| nM | apicidin |
| | trapoxin |
| | TPX-HA analog |
| nM - µM | TSA |
| | depsipeptide |
| | SAHA |
| | scriptaid |
| | DBCH |
| | suberic bis-hydroxamic acid |
| | pyroxamide |
| | m-carboxy cinnamic acid bis-hydroxamic |
| | acid |
| µM | oxamflatin |
| | benzamide (MS-27-275) |
| | depudecin |
| mM | butyric acid |
| | phenylbutyrate |

Dosage ranges appropriate for practice of the present invention are in the range of about 10 nm to about 500 mM, preferably about 2 µM to about 280 mM. Dosages adjusted for body weight appropriate for practice of the present invention range from about 1 milligram per kilogram body weight to 1,000 milligram/kilogram, preferably about 25 mg/kg to about 100 mg/kg.

For this invention the deacetylase inhibitor will be administered at dosages and for periods of time effective to reduce, ameliorate or eliminate the symptoms of the disease or pathological condition. Dose regimens may be adjusted for purposes of improving the therapeutic or prophylactic response of the compound. For example, several divided doses may be administered daily, one dose, or cyclic administration of the compounds to achieve the desired therapeutic result. A single deacetylase inhibitor may be administered or combinations of various deacetylase inhibitors may be administered. Agents that improve the solubility of these compounds could also be added.

The deacetylase inhibitors can be formulated with one or more adjuvants and/or pharmaceutically acceptable carriers according to the selected route of administration. The addition of gelatin, flavoring agents, or coating material can be used for oral applications. For solutions or emulsions in general, carriers may include aqueous or alcoholic/aqueous solutions, emulsions or suspensions, including saline and buffered media. Parenteral vehicles can include sodium chloride and potassium chloride, among others. In addition, intravenous vehicles can include fluid and nutrient replenishers, electrolyte replenishers and the like.

Preservatives and other additives can also be present. For example, antimicrobial, antioxidant, chelating agents, and inert gases can be added (see, generally, Remington's Pharmaceutical Sciences, 16th Edition, Mack, (1980)).

***Drosophila* models of polyglutamine pathogenesis useful for testing potential therapeutic agents**. In addition to providing a method of treating various neurological and psychiatric diseases by administration of deacetylase inhibitors, the present invention is also directed to a transgenic fly model useful for testing potential therapeutic agents. As discussed above, the present inventors have developed a transgenic fly stock expressing Httex 1 pQ93, and have shown that expression of the transgene results in cell lethality and neurodegeneration as measured by the progressive loss of rhabdomeres. The present inventors have also demonstrated that lethality and neurodegeneration in the transgenic fly can be suppressed by administration of deacetylase inhibitors such as SAHA and butyrate. Other indicators of polyglutamine pathogenesis, including loss of motor function, decreased viability (e.g., premature death of the fly prior to completing development into the adult form), and early death of the adult fly, may also be evaluated in the transgenic fly stock.

The transgenic fly stock thus provides a useful tool for assessing the therapeutic potential of candidate drugs for treating the neurodegenerative pathology of Huntington's disease and other polyglutamine-related diseases or disorders, wherein various candidate drugs are fed to or otherwise tested on transgenic flies to evaluate suppression of lethality and/or neurodegeneration.

The present invention is demonstrated more fully by the following prophetic examples, which are not intended to be limiting in any way.
**Example 1.** Suberoylanilide hydroxamic acid (SAHA): administration to treat polyglutamine-related neurodegeneration. An exemplary embodiment for treating polyglutamine-related neurodegeneration in accordance with practice of principles of this invention comprises parenterally administering a therapeutically effective dosage of SAHA, about 500 nm to about 500 µm, in an aqueous solution to the patient.
**Example 2.** Sodium phenylbutyrate: administration to treat schizophrenia. An exemplary embodiment for treating schizophrenia in accordance with practice of principles of this invention comprises orally administering tablets comprising 500 mg powdered sodium phenylbutyrate three times per day to the patient.
**Example 3.** Pyroxamide: administration to treat Huntington's disease. An exemplary embodiment for treating Huntington's disease in accordance with practice of principles of this invention comprises parenterally administering a therapeutically effective dosage of pyroxamide, ranging from about 2 nm to about 500 µm, in an aqueous solution to the patient.
**Example 4.** Suberoylanilide hydroxamic acid (SAHA): administration to treat polyglutamine-related neurodegeneration. An exemplary embodiment for treating polyglutamine-related neurodegeneration in accordance with practice of principles of this invention comprises parenterally administering 25 mg SAHA per kg body weight in an aqueous solution to the patient.
**Example 5.** Depsipeptide: Administration to treat Huntington's Disease. An exemplary embodiment for treating Huntington's Disease in accordance with practice of principles of this invention comprises parenterally administering 100 mg depsipeptide per kg body weight in an aqueous solution to the patient.

## Claims

1. The use of histone deacetylase inhibitor for the preparation of a medicament for the treatment of a polyglutamine-expansion-related neurodegenerative disease.

2. The use as set forth in claim 1, wherein the disease is selected from the group consisting of: Huntington's disease, Kennedy's disease, spinocerebellar ataxia diseases, DRPLA, and Machado-Joseph disease.

3. The use as set forth in claim 1, wherein the histone deacetylase inhibitor is selected from the group consisting of SAHA, butyrate, pyroxamide, depsipeptide, MS-275, and derivatives thereof.

4. The use as set forth in claim 1, wherein the histone deacetylase inhibitor is a Sir2 inhibitor or a derivative thereof.

5. The use as set forth in claim 1, wherein the histone deacetylase inhibitor is a SAHA or a derivative thereof.

6. The use as set forth in claim 1, wherein the histone deacetylase inhibitor is a sodium phenylbutyrate or a derivative thereof.

## Patentansprüche

1. Verwendung eines Histondeacetylase-Inhibitors zur Herstellung eines Medikaments zur Behandlung einer mit einer Polyglutamin-Expansion zusammenhängenden neurodegenerativen Erkrankung.

2. Verwendung wie in Anspruch 1 dargelegt, wobei die Erkrankung aus der Gruppe ausgewählt ist, bestehend aus: Chorea Huntington, Kennedy-Syndrom, Erkrankungen mit spinozerebellärer Ataxie, DRPLA, und Machado-Joseph-Syndrom.

3. Verwendung wie in Anspruch 1 dargelegt, wobei der Histondeacetylase-Inhibitor aus der Gruppe ausgewählt ist, bestehend aus SAHA, Butyrat, Pyroxamid, Depsipeptid, MS-275 und Derivaten davon.

4. Verwendung wie in Anspruch 1 dargelegt, wobei es sich bei dem Histondeacetylase-Inhibitor um einen Sir2-Inhibitor oder ein Derivat davon handelt.

5. Verwendung wie in Anspruch 1 dargelegt, wobei es sich bei dem Histondeacetylase-Inhibitor um eine SAHA oder ein Derivat davon handelt.

6. Verwendung wie in Anspruch 1 dargelegt, wobei es sich bei dem Histondeacetylase-Inhibitor um ein Natriumphenylbutyrat oder ein Derivat davon handelt.

## Revendications

1. Utilisation de l'inhibiteur de l'histone désacétylase pour la préparation d'un médicament pour le traitement d'une maladie neurodégénérative liée à une expansion de la polyglutamine.

2. Utilisation selon la revendication 1, dans laquelle la maladie est choisie dans le groupe constitué par la maladie d'Huntington, la maladie de Kennedy, les maladies ataxies spino-cérébelleuses, la DRPLA et la maladie de Machado-Joseph.

3. Utilisation selon la revendication 1, dans laquelle l'inhibiteur de l'histone désacétylase est choisi dans le groupe constitué par le SAHA, un butyrate, le pyroxamide, un depsipeptide, le MS-275 et les dérivés de ceux-ci.

4. Utilisation selon la revendication 1, dans laquelle l'inhibiteur de l'histone désacétylase est un inhibiteur Sir2 ou un dérivé de celui-ci.

5. Utilisation selon la revendication 1, dans laquelle l'inhibiteur de l'histone désacétylase est un SAHA ou un dérivé de celui-ci.

6. Utilisation selon la revendication 1, dans laquelle l'inhibiteur de l'histone désacétylase est un phénylbutyrate de sodium ou un dérivé de celui-ci.
